# EUROPEAN PATENT APPLICATION

(11) **EP 3 995 565 A1**
(43) Date of publication of application: **11.05.2022**
(21) Application number: 20834585.0
(22) Date of filing: 19.06.2020
(51) Int. Cl.: C12M 1/34, C12M 1/00

(54) **MOLECULAR DETECTION SYSTEM**

(30) Priority: 01.07.2019 CN 201921010541 U; 01.07.2019 CN 201921024226 U; 01.07.2019 CN 201921011139 U; 01.07.2019 CN 201921010538 U; 01.07.2019 CN 201921012340 U; 01.07.2019 CN 201910585275; 09.10.2019 CN 201910953746; 09.10.2019 CN 201921681143 U; 09.10.2019 CN 201921681163 U; 09.10.2019 CN 201921681583 U; 09.10.2019 CN 201921681136 U; 09.10.2019 CN 201921681140 U
(71) Applicant: Shenyi Biotech (Hangzhou) Co., Ltd., Hangzhou, Zhejiang 311121 (CN)
(72) Inventor: XU, Qiang, Hangzhou, Zhejiang 311121 (CN); CUI, Xiangmin, Hangzhou, Zhejiang 311121 (CN)
(74) Representative: Habermann, Hruschka & Schnabel
(86) International application number: PCT/CN2020/097140
(87) International publication number: WO 2021/000749

(57) **Abstract**

Provided is a molecular detection system and a microfluidic kit adapted for the molecular detection system. The molecular detection system includes: a substrate; and a fixing apparatus; a driving apparatus; a detection apparatus; and a temperature control apparatus that are arranged on the substrate, the fixing apparatus is adapted to fix a container containing a sample to be detected; the driving apparatus is adapted to drive the container to preprocess the sample to be detected; the detection apparatus is adapted to detect the sample to be detected that has undergone the preprocessing; and the temperature control apparatus is adapted to regulate a temperature of the sample to be detected. The microfluidic kit includes: a box body having a reagent zone, a sample zone, and a reaction zone inside; and a microfluidic kit chip including a microfluidic pipe with a piercing member provided inside and facing a liquid outlet, the microfluidic kit chip being connected respectively to the reagent zone, the sample zone and the reaction zone through the microfluidic pipe.

## Description

### FIELD

The present disclosure relates to the field of molecular biology, in particular to a molecular detection system.

### BACKGROUND

Biomolecules and other analytes can be detected by selective or specific probes that can react with them. With the development of molecular biology, the detection of protein, DNA, RNA and other biological molecules has become an effective means of disease diagnosis and prediction. The advantages of molecule diagnostic technology, such as low sample consumption, accurate diagnostic results, high sensitivity, and high throughput, have enabled the technology to develop rapidly in modern medicine.

Currently in molecule diagnostic technology, the most widely used detection method is fluorescent labeling detection technology. This technology uses fluorescent molecular groups as markers, and connects fluorescent markers to probe molecules or target molecules through chemical modification. The detection instrument can realize the quantitative detection of specific molecules by irradiating laser light having a specific wavelength on the sample to be detected, and detecting the corresponding fluorescence signal intensity. However, the existing molecular detection methods still need to be improved.

### SUMMARY

The present disclosure is based on Applicant's discovery of the following facts and problems:

Existing molecular detection instruments generally do not directly detect samples, and a lot of auxiliary work needs to be done before detection. For example, according to the specific conditions of the sample, the nucleic acid in the sample needs to be purified, the required molecule fragments are extracted, the corresponding detection reagents are prepared, and finally the sample and the detection reagents are mixed and placed in the detection instrument for detection. The auxiliary work is cumbersome and needs to be done with different instruments or tools, so that molecular detection must be performed by professionals in a standardized laboratory environment, with limited application scenarios, long detection time, and low efficiency.

In view of this, the present disclosure proposes a molecular detection system. The molecular detection system can perform fully automated and rapid detection of samples to be detected.

In one aspect of the present disclosure, the present disclosure provides a molecular detection system. According to an embodiment of the present disclosure, the molecular detection system includes: a substrate; a fixing apparatus; a driving apparatus; a detection apparatus; and a temperature control apparatus, the fixing apparatus, the driving apparatus, the detection apparatus, and the temperature control apparatus are arranged on the substrate, the fixing apparatus is adapted to fix a container containing a sample to be detected; the driving apparatus is adapted to drive the container to preprocess the sample to be detected; the detection apparatus is adapted to detect the sample to be detected that has undergone the preprocessing; and the temperature control apparatus is adapted to regulate a temperature of the sample to be detected.

According to the molecular detection system of the embodiment of the present disclosure, the container containing the sample to be detected is fixed on the fixing apparatus. Under the action of the driving apparatus, the nucleic acid extraction and PCR reaction in the sample is completed while the sample to be detected flows in the container, thereby obtaining samples adapted for detection by the detection apparatus. When the driving apparatus drives the sample to be detected to flow in the container, the temperature control apparatus can heat a local part of the fixing apparatus, so as to realize the extraction of nucleic acids in the sample and the PCR reaction. While the PCR reaction is in progress, the detection apparatus can perform real-time fluorescence measurement on the amplified product, and obtain the detection result based on the data of the fluorescence signal change. As a result, the molecular detection system of the present disclosure integrates auxiliary work such as purification and extraction of sample nucleic acids into one device, and completes molecular detection in a fully automated manner, which solves the problems of the traditional molecular detection methods, such as limited application scenarios and low efficiency caused by restrictions in experimental conditions and staff.

In addition, the molecular detection system according to the above embodiment of the present disclosure may further have the following additional technical features:

In some embodiments of the present disclosure, the substrate includes a first base, a support, and a shaft sleeve, and the first base, the support, and the shaft sleeve are connected to each other.

In some embodiments of the present disclosure, the fixing apparatus includes a slot, a first motor, a first nut, a first clamping member, a second clamping member and a connecting rod; the first motor is connected to the first clamping member by the first nut, the first clamping member is connected to the second clamping member by the connecting rod, and the slot is provided between the first clamping member and the second clamping member.

In some embodiments of the present disclosure, the molecular detection system further includes: a first compression spring disposed between the slot and the second clamping member.

In some embodiments of the present disclosure, the molecular detection system further includes: a first heat dissipation component provided under the slot.

In some embodiments of the present disclosure, the driving apparatus includes a second motor, a third motor, a thimble, and a push rod, the second motor is connected to the thimble, and the third motor is connected to the push rod.

In some embodiments of the present disclosure, the molecular detection system further includes: a linear guide rail and a sliding block, and the push rod is arranged on the linear guide rail through the sliding block.

In some embodiments of the present disclosure, the detection apparatus includes an optical path detection unit, and the temperature control apparatus includes a chip temperature cycle control unit.

In some embodiments of the present disclosure, one end of the detection apparatus is connected to the slot through the chip temperature cycle control unit.

In some embodiments of the present disclosure, the molecular detection system further includes a control system, and the control system is connected respectively to the fixing apparatus, the driving apparatus, the detection apparatus, and the temperature control apparatus, and is adapted to control the fixing apparatus, the driving apparatus, the detection apparatus, and the temperature control apparatus to operate collaboratively to detect the sample to be detected.

In some embodiments of the present disclosure, the molecular detection system further includes: a sliding apparatus connected to the driving apparatus and the fixing apparatus, and adapted to hold the container in a relatively fixed position.

In some embodiments of the present disclosure, the sliding apparatus includes: a second base; a first guide rod movably inserted in a fixed limit through hole located on one end of the second base; a second guide rod movably inserted in a movable limit through hole on the other end of the second base; a first connecting bracket having a third through hole provided thereon, wherein an upper end of the first guide rod and an upper end of the second guide rod are connected to two ends of the first connecting bracket, respectively; a second connecting bracket, wherein a lower end of the first guide rod and a lower end of the second guide rod are connected to two ends of the second connecting bracket, respectively; and a first screw motor arranged on the second base, wherein a screw in the first screw motor is perpendicular to the second base and extends through the third through hole, the screw is adapted to drive the first connecting bracket to move and jointly move the first guide rod, the second guide rod and the second connecting bracket.

In some embodiments of the present disclosure, a diameter of the first guide rod is 0.1mm to 0.3mm smaller than an inner diameter of the fixed limit through hole, and a diameter of the second guide rod is 0.5mm to 2mm smaller than an inner diameter of the movable limit through hole.

In some embodiments of the present disclosure, the upper end of the first guide rod and the upper end of the second guide rod are connected to the two ends of the first connecting bracket through bolts, respectively, and the lower end of the first guide rod and the lower end of the second guide rod are connected to the two ends of the second connecting bracket through bolts, respectively.

In some embodiments of the present disclosure, the screw is connected to the third through hole through screw threads.

In some embodiments of the present disclosure, the sliding apparatus further includes: a first screw nut fixedly connected at the third through hole and matched with and connected to the screw.

In some embodiments of the present disclosure, the first connecting bracket is movable within an interval of 5mm to 55mm from the second base.

In some embodiments of the present disclosure, a lubricating layer is formed on a surface of each of the fixed limit through hole and the movable limit through hole.

In some embodiments of the present disclosure, the lubricating layer is a lubricating oil layer or a graphite layer, and a thickness of the lubricating layer is 0.01mm to 1mm.

In some embodiments of the present disclosure, the screw in the first screw motor is a trapezoidal screw, a ball screw or a planetary ball screw, and a motor in the first screw motor is a direct current motor, a stepper motor or a servo motor.

In some embodiments of the present disclosure, the molecular detection system further includes: an optical path imaging automatic adjustment apparatus connected to the detection apparatus, and including a photoelectric sensor and a convex lens, wherein the optical path imaging automatic adjustment apparatus is adapted to automatically adjust a distance between the photoelectric sensor and the convex lens.

In some embodiments of the present disclosure, the optical path imaging automatic adjustment apparatus further includes: a third base, an adjusting bracket, and a second screw motor. The photoelectric sensor is fixed on the third base, the adjusting bracket is movably arranged on the third base, the convex lens is provided on the adjusting bracket, the distance between the photoelectric sensor and the convex lens is adjusted by moving the adjusting bracket, and a second screw motor fixed on the third base, wherein a screw in the second screw motor passes through the adjusting bracket and drives the adjusting bracket to move.

In some embodiments of the present disclosure, the adjusting bracket is threadedly connected to the screw.

In some embodiments of the present disclosure, the optical path imaging automatic adjustment apparatus further includes: a second screw nut fixedly connected to the adjusting bracket and matched with and connected to the screw.

In some embodiments of the present disclosure, the adjusting bracket is movable within a distance smaller than or equal to 30mm.

In some embodiments of the present disclosure, the distance between the photoelectric sensor and the convex lens is 30mm to 60mm.

In some embodiments of the present disclosure, a plurality of protrusions that abuts against an inner wall of the third base is provided on a peripheral wall of the adjusting bracket.

In some embodiments of the present disclosure, the second screw motor and the photoelectric sensor are intelligently and jointly controlled through a CPU end.

In some embodiments of the present disclosure, the screw in the second screw motor is a trapezoidal screw, a ball screw, or a planetary ball screw, and a motor in the second screw motor is a direct current motor, a stepper motor or a servo motor.

In some embodiments of the present disclosure, the slot in the molecular detection system includes: a housing, wherein an upper part of the housing is open and defines an accommodating space inside; at least one positioning hole defined in a side portion of the housing; at least one positioning pin each having one end extending into one of the at least one positioning hole; a heating assembly arranged in the accommodating space; and a second compression spring arranged at a bottom of the accommodating space and connected to the heating assembly.

In some embodiments of the present disclosure, the slot further includes a fixing screw extending through the via hole into the accommodating space to be connected to the heating assembly, and the second compression spring is sleeved on the fixing screw.

In some embodiments of the present disclosure, the at least one positioning hole includes a plurality of positioning holes, the at least one positioning pin includes a plurality of positioning pins, and the plurality of positioning holes is arranged in one-to-one correspondence with the plurality of positioning pins.

In some embodiments of the present disclosure, the end of the positioning pin extending into the positioning hole is conical.

In some embodiments of the present disclosure, the heating assembly includes a heating sheet and a heat conduction block that are connected to each other, and the heat conduction block is connected to the second compression spring and the fixing screw.

In some embodiments of the present disclosure, a heating chamber is formed on the heat conduction block.

In some embodiments of the present disclosure, a lower part of the heat conduction block is provided with a limiting hole, and the fixing screw extends into the limiting hole.

In some embodiments of the present disclosure, a diameter of the via hole is larger than a diameter of the fixing screw.

In another aspect of the present disclosure, the present disclosure provides a microfluidic kit adapted for the molecular detection system of the above embodiment. According to an embodiment of the present disclosure, the microfluidic kit includes: a box body having a reagent zone, a sample zone, and a reaction zone inside; a microfluidic kit chip including a microfluidic pipe with a piercing member provided inside and facing a liquid outlet, wherein the microfluidic kit chip is connected respectively to the reagent zone, the sample zone and the reaction zone through the microfluidic pipe.

The microfluidic kit of the above embodiment of the present disclosure can not only introduce the sample to be detected in the sample zone and the reagent in the reagent zone (if the reaction zone has a freeze-dried powder reagent, the reagent zone can also store a diluent) into the reaction zone for reaction through the microfluidic pipe, thereby realizing the detection of the sample, and there is no need to set additional external parts such as elastic valves to realize the disconnection and connection of the pipe.

In some embodiments of the present disclosure, the microfluidic pipe used for the microfluidic kit chip further includes: a pipe and a sealing member. The pipe has an end portion made of an elastic material, the liquid outlet is provided in a side wall of the end portion of the pipe, the sealing film is arranged at the liquid outlet and seals the liquid outlet; the piercing member has a tip portion, and is located on an inner wall of the end portion of the pipe opposed to the liquid outlet, and the tip portion faces the liquid outlet and is configured to pierce through the sealing film when the end portion is squeezed under an external force. Therefore, the microfluidic pipe can realize the dual functions of sealing the microfluidic pipe before use and unsealing during use, and the sealing of the pipe can be realized without adding external pressure parts when storing raw materials.

In some embodiments of the present disclosure, the piercing member and the liquid outlet are provided on a same diameter of the pipe. Therefore, it is easier to pierce the sealing film when a force is applied to the end portion of the pipe.

In some embodiments of the present disclosure, the sealing film is a tin foil paper, a laminating film or a kraft paper. This can effectively prevent the raw materials stored in the microfluidic pipe from seeping out through permeation.

In some embodiments of the present disclosure, the sealing film has a thickness in a range from 0.01mm to 0.2mm. This not only facilitates the sealing of the liquid outlet, but also reduces the difficulty in piercing the sealing film by the piercing member.

In some embodiments of the present disclosure, a tip of the tip portion has a tapered structure, the tapered structure being a cone or a polygonal pyramid. This can further facilitate the piercing of the sealing film.

In some embodiments of the present disclosure, the tip portion is formed by a triangular surface, wherein one side of the triangular surface is provided on the inner wall of the end portion of the pipe opposed to the liquid outlet and an apex angle corresponding to the side of the triangular surface forms a tip of the tip portion. This can further facilitate the piercing of the sealing film.

In some embodiments of the present disclosure, the side of the triangular face provided on the inner wall of the end portion of the pipe is straight line-shaped, V-shaped, arc-shaped or U-shaped.

In some embodiments of the present disclosure, the piercing member and the pipe are integrally formed into one piece. Therefore, the structure of the microfluidic pipe can be further simplified, and it further facilitates the piercing of the sealing film.

In some embodiments of the present disclosure, the pipe includes a first plate and an elastic plate, a lower surface of the first plate has a groove, the liquid outlet is provided in the groove, the elastic plate is arranged on the lower surface of the first plate and seals the groove so that the groove forms a microfluidic channel, the piercing member is located in the microfluidic channel and at a position opposed to the liquid outlet, the piercing member is connected to the lower surface of the first plate through an elastic member, and the piercing member is configured to pierce through the sealing film when the elastic plate is squeezed under an external force.

The additional aspects and advantages of the present disclosure will be partly given in the following description, and part of them will become apparent from the following description, or be understood through the practice of the present disclosure.

### BRIEF DESCRIPTION OF DRAWINGS

The accompanying drawings are used to provide a further understanding of the present disclosure, constitute a part of the specification, and are used to explain the present disclosure together with the following specific embodiments, but does not constitute a limitation to the present disclosure. In the accompanying drawings:
FIG. 1 is a structural schematic diagram of a molecular detection system according to an embodiment of the present disclosure.
FIG. 2 is a structural schematic diagram of a molecular detection system according to an embodiment of the present disclosure.
FIG. 3 is a structural schematic diagram from another perspective of a molecular detection system according to an embodiment of the present disclosure.
FIG. 4 is a structural schematic diagram from another perspective of a molecular detection system according to an embodiment of the present disclosure.
FIG. 5 is a structural schematic diagram from another perspective of a molecular detection system according to an embodiment of the present disclosure.
FIG. 6 is a structural schematic diagram of some components of a fixing apparatus, a driving apparatus and a temperature control apparatus in a molecular detection system according to an embodiment of the present disclosure.
FIG. 7 is a structural schematic diagram of some components of a detection apparatus in a molecular detection system according to an embodiment of the present disclosure.
FIG. 8 is a structural schematic diagram from another perspective of some components of a detection apparatus in a molecular detection system according to an embodiment of the present disclosure.
FIG. 9 is a structural schematic diagram of some components of a fixing apparatus and a driving apparatus in a molecular detection system according to an embodiment of the present disclosure.
FIG. 10 is a structural schematic diagram from another perspective of some components of a fixing apparatus and a driving apparatus in a molecular detection system according to an embodiment of the present disclosure.
FIG. 11 is a structural schematic diagram from another perspective of some components of a fixing apparatus and a driving apparatus in a molecular detection system according to an embodiment of the present disclosure.
FIG. 12 is a structural schematic diagram of a microfluidic kit according to an embodiment of the present disclosure.
FIG. 13 is a structural schematic diagram of a sliding apparatus in a molecular detection system according to an embodiment of the present disclosure.
FIG. 14 is a structural schematic diagram of a sliding apparatus in a molecular detection system according to another embodiment of the present disclosure.
FIG. 15 is a cross-sectional view of a second base in a molecular detection system along a longitudinal direction according to an embodiment of the present disclosure.
FIG. 16 is a structural schematic diagram of a sliding apparatus in a molecular detection system according to another embodiment of the present disclosure.
FIG. 17 is a structural schematic diagram of a sliding apparatus in a molecular detection system according to another embodiment of the present disclosure.
FIG. 18 is a structural schematic diagram of a sliding apparatus in a molecular detection system according to another embodiment of the present disclosure.
FIG. 19 is a structural schematic diagram of an optical path imaging automatic adjustment apparatus in a molecular detection system according to an embodiment of the present disclosure.
FIG. 20 is a structural diagram of a limiting relationship between an adjusting bracket of an optical path imaging automatic adjustment apparatus and a third base in a molecular detection system according to an embodiment of the present disclosure.
FIG. 21 is a structural schematic diagram of a slot in a molecular detection system according to an embodiment of the present disclosure;
FIG. 22 is a structural schematic diagram from another perspective of a slot in a molecular detection system according to an embodiment of the present disclosure;
FIG. 23 is a structural schematic diagram of a kit adapted for a slot in a molecular detection system of an embodiment of the present disclosure;
FIG. 24 is a structural schematic diagram of fit installation of a slot and a kit in a molecular detection system according to an embodiment of the present disclosure;
FIG. 25 is a structural schematic diagram from another perspective of fit installation of a slot and a kit in a molecular detection system according to an embodiment of the present disclosure.
FIG. 26 is a structural diagram of a microfluidic kit according to an embodiment of the present disclosure.
FIG. 27 is a structural diagram of a microfluidic pipe according to an embodiment of the present disclosure.
FIG. 28 is a cross-sectional view of a microfluidic pipe in a direction perpendicular to the pipe according to an embodiment of the present disclosure.
FIG. 29 is a cross-sectional view of a microfluidic pipe in a direction parallel to the pipe according to an embodiment of the present disclosure.
FIG. 30 is a top view of a piercing member according to an embodiment of the present disclosure.
FIG. 31 is a structural diagram of a microfluidic pipe according to another embodiment of the present disclosure.
FIG. 32 is a structural diagram of a tip portion of a microfluidic pipe according to another embodiment of the present disclosure.
FIG. 33 is a cross-sectional view of a microfluidic pipe in a direction perpendicular to the pipe according to another embodiment of the present disclosure.
FIG. 34 is a cross-sectional view of a microfluidic pipe in a direction parallel to thejhujhhjh pipe according to another embodiment of the present disclosure.
FIG. 35 is a top view of a piercing member according to another embodiment of the present disclosure.
FIG. 36 is a perspective view of a partial composition of a microfluidic pipe according to an embodiment of the present disclosure.
FIG. 37 is a perspective view of a microfluidic pipe according to an embodiment of the present disclosure.
FIG. 38 is a perspective view of a partial composition of a microfluidic pipe according to still another embodiment of the present disclosure.
FIG. 39 is a perspective view of a microfluidic pipe according to still another embodiment of the present disclosure.

### DESCRIPTION OF EMBODIMENTS

The embodiments of the present disclosure will be described in detail below, and examples thereof are as illustrated in the accompanying drawings, throughout which same or similar elements, or elements having same or similar functions, are denoted by same or similar reference numerals. The embodiments described below with reference to the drawings are illustrative, and are intended to explain rather than limit the present disclosure.

In the description of the present disclosure, it should be understood that the orientation or position relationship indicated by the terms "center", "longitudinal", "transverse", "length", "width", "thickness", "on", "under", "front", "rear", "left", "right", "vertical", "horizontal", "top", "bottom", "inner", and "outer", "clockwise", "counterclockwise", "axial", "radial", "circumferential", etc. is based on the orientation or position relationship shown in the drawings, and is only for the convenience for describing the present disclosure and simplifying the description, rather than indicating or implying that the pointed device or element must have a specific orientation, or be constructed and operated in a specific orientation, and therefore cannot be understood as a limitation to the present disclosure.

In addition, the terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present disclosure, "plurality" means at least two, such as two, three, etc., unless otherwise specifically defined.

In the present disclosure, unless otherwise clearly specified and limited, terms such as "install", "connect", "connected to", "fix" and the like should be understood in a broad sense. For example, it may be a fixed connection or a detachable connection or connection as one piece; mechanical connection or electrical connection; direct connection or indirect connection through an intermediate; internal connection of two components or the interaction relationship between two components, unless otherwise clearly limited. For those of ordinary skill in the art, the specific meaning of the above-mentioned terms in the present disclosure can be understood according to specific circumstances.

In the present disclosure, unless expressly stipulated and defined otherwise, the first feature "on" or "under" the second feature may mean that the first feature is in direct contact with the second feature, or the first and second features are in indirect contact with each other through an intermediate. Moreover, the first feature "above" the second feature may mean that the first feature is directly above or obliquely above the second feature, or simply mean that the level of the first feature is higher than that of the second feature. The first feature "under" the second feature may mean that the first feature is directly below or obliquely below the second feature, or simply mean that the level of the first feature is lower than that of the second feature.

Applicant found in the research of molecular detection instruments that the existing molecular detection instruments generally do not directly detect samples, and a lot of auxiliary work needs to be done before detection. For example, according to the specific conditions of the sample, the nucleic acid in the sample needs to be purified, the required molecule fragments are extracted, the corresponding detection reagents are prepared, and finally the sample and the detection reagents are mixed and placed in the detection instrument for detection. The auxiliary work is cumbersome and needs to be done with different instruments or tools, so that molecular detection must be performed by professionals in a standardized laboratory environment, with limited application scenarios, long detection time, and low efficiency.

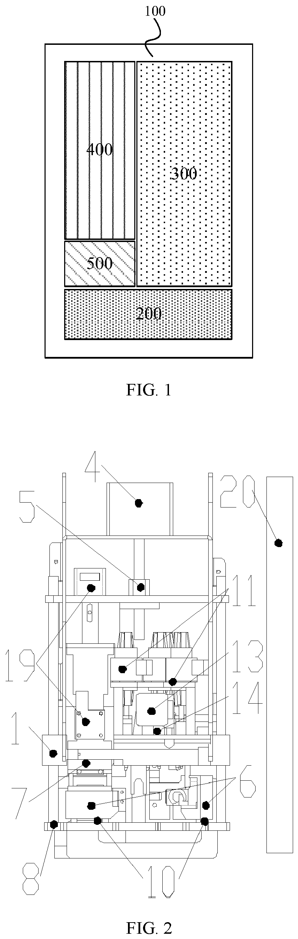

In view of this, in one aspect of the present disclosure, the present disclosure proposes a molecular detection system. According to an embodiment of the present disclosure, referring to FIG. 1, the molecular detection system includes: a substrate 100; a fixing apparatus 200; a driving apparatus 300; a detection apparatus 400; and a temperature control apparatus 500. The a fixing apparatus 200, the driving apparatus 300, the detection apparatus 400, and the temperature control apparatus 500 are arranged on the substrate 100. The fixing apparatus 200 is adapted to fix a container containing a sample to be detected; the driving apparatus 300 is adapted to drive the container to preprocess the sample to be detected; the detection apparatus 400 is adapted to detect the sample to be detected that has undergone the preprocessing; and the temperature control apparatus 500 is adapted to regulate a temperature of the sample to be detected.

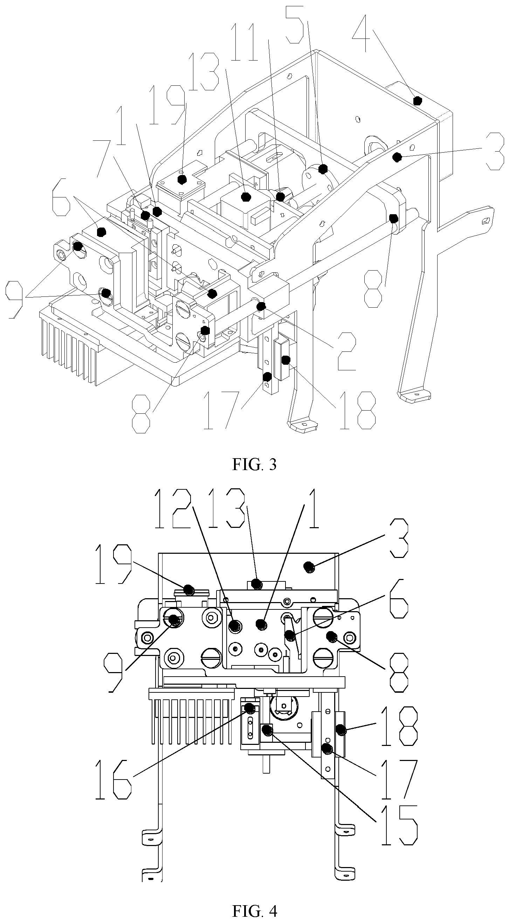

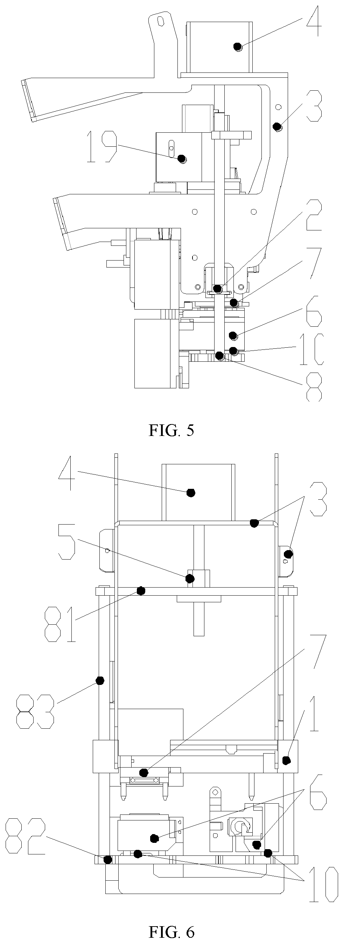

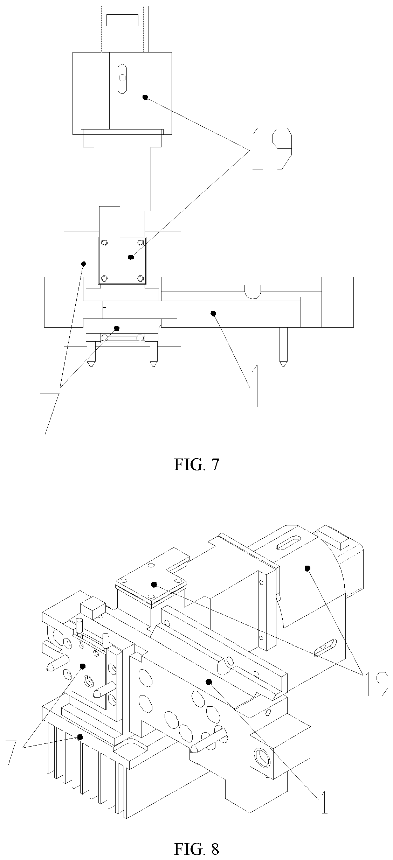

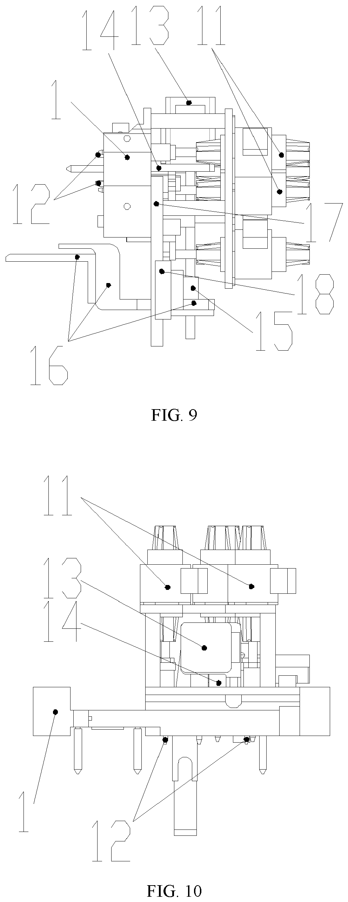

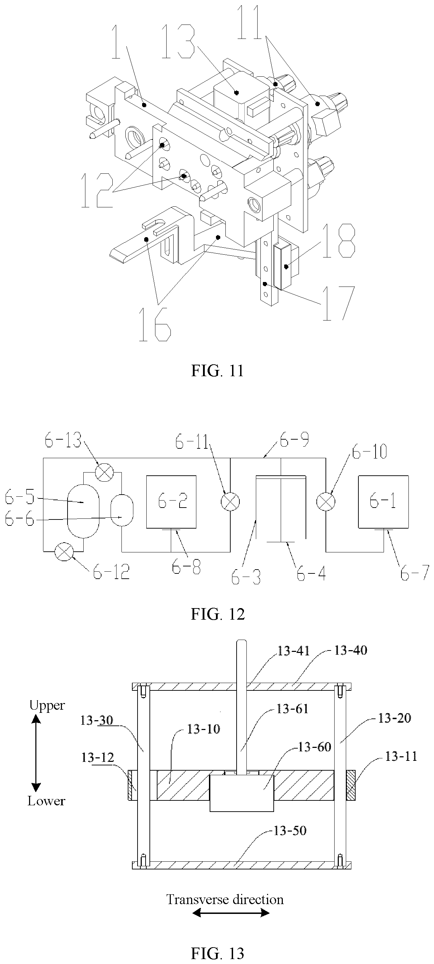

The molecular detection system of the present disclosure will be further described in detail below with reference to FIGS. 1 to 12. FIGS. 2 to 5 are structural schematic diagrams from different perspectives of the molecular detection system. FIG. 6 shows in detail a relative positional relationship of some components in the fixing apparatus 200, the driving apparatus 300, and the temperature control apparatus 500. FIGS. 7 and 8 respectively show in detail a relative positional relationship of some components in the detection apparatus 400 from different perspectives. FIGS. 9 to 11 respectively show in detail a relative positional relationship of some components in the fixing apparatus 200 and the driving apparatus 300 from different perspectives. FIG. 12 is a structural schematic diagram of the container containing a sample to be detected.

The specific structure of the substrate 100 is not particularly limited, as long as it can provide support for the fixing apparatus 200, the driving apparatus 300, the detection apparatus 400, and the temperature control apparatus 500, so that each apparatus can operate stably. According to an embodiment of the present disclosure, referring to FIGS. 2 to 6, the substrate 100 includes a first base 1, a support 2 and a shaft sleeve 3, and the first base 1, the support 2 and the shaft sleeve 3 are connected to each other and together form a support structure of the molecular detection system, which provides support for the fixing apparatus 200, the driving apparatus 300, the detection apparatus 400 and the temperature control apparatus 500.

The fixing apparatus 200 is adapted for fixing the container containing the sample to be detected, and can ensure that the container containing the sample to be detected is in a relatively fixed position during the detection process, thereby ensuring that the preprocessing and the detection processes of the sample to be detected are carried out stably. According to an embodiment of the present disclosure, referring to FIGS. 2 to 6, the fixing apparatus includes a slot 6, a first motor 4, a first nut 5 and a clamping device 8. The clamping device 8 includes a first clamping member 81, a second clamping members 82 and a connecting rod 83. The first motor 4 is connected to the first clamping member 81 by a first nut 5, the first clamping member 81 is connected to the second clamping member 82 by the connecting rod 83, and the slot 6 is provided between the first clamping member 81 and the second clamping member 82. As shown in FIGS. 2 to 6, the slot 6 is provided on the first base 1, the first motor 4 is provided on the shaft sleeve 3, and the connecting rod 83 in the clamping device 8 extends through the support 2 to be connected to the first clamping member 81 and the second clamping member 82. During the detection process, the container containing the sample to be detected is placed in the slot 6, and as the first motor 4 drives the first nut 5 to move, the first clamping member 81 and the second clamping member 82 in the clamping device 8 can clamp the container in the slot 6 tight so as to ensure that the position of the container is relatively fixed during the detection process.

According to an embodiment of the present disclosure, the molecular detection system may further include: a first compression spring 10. The first compression spring 10 is disposed between the slot 6 and the second clamping member 82 and is loosely connected between the slot 6 and the second clamping member 82 by a screw 9. Therefore, during the movement of the clamping device 8, the container in the slot 6 is clamped tight under the action of the first compression spring 10.

According to an embodiment of the present disclosure, the molecular detection system may further include: a first heat dissipation component 61 provided under the slot 6. Specifically, the first heat dissipation component 61 may be installed on the first base 1 and under the slot 6. By providing the first heat dissipation component 61 under the slot 6, it is more conducive to the heat dissipation of the container containing the sample to be detected, thereby facilitating the temperature control apparatus to regulate the temperature of the container. The specific type of the first heat dissipation component 61 is not particularly limited. For example, a fin type heat sink, a plate type heat sink, etc. may be adopted.

The driving apparatus 300 is adapted to drive the container containing the sample to be detected to preprocess the sample to be detected. The preprocessing includes extraction of nucleic acid from the sample to be detected and PCR reaction. In order to facilitate understanding, the structure and working principle of the container containing the sample to be detected will be described in detail below. It should be noted that in this specification, "a container containing a sample to be detected" is also referred to as a "kit" or a "microfluidic kit".

Referring to FIG. 12, the kit includes a sample chamber 6-1, a dilution chamber 6-2, an injection chamber 6-3, a piston 6-4, a PCR chamber 6-5, a buffer chamber 6-6, a sample sealing film 6-7, and a dilution sealing film 6-8, a microfluidic pipe 6-9, a sample control valve 6-10, a dilution control valve 6-11, a first PCR control valve 6-12, and a second PCR control valve 6-13, and respective units are connected by the microfluidic pipe 6-9 to form a connected loop.

In an initial state, the sample chamber 6-1 contains a lysis raw material in the form of freeze-dried powder, the PCR chamber 6-5 contains a reverse transcriptase and PCR raw materials in the form of freeze-dried powder, and the dilution chamber 6-2 contains an appropriate diluent. A portion of the sample chamber 6-1 connected to the microfluidic pipe 6-9 and a portion of the dilution chamber 6-2 connected to the microfluidic pipe 6-9 are sealed with a sample sealing film 6-7 and a dilution sealing film 6-8, respectively, so that the lysis raw material in the sample chamber 6-1 is isolated from the diluent in the dilution chamber 6-2 and the reverse transcriptase and PCR raw materials in the PCR chamber 6-5. The piston 6-4 is at a top end of the injection chamber 6-3 (the injection chamber is in a state of being fully filled by the piston).

When the sample is added to the sample chamber 6-1, the microfluidic kit starts to work. Firstly, the sample sealing film 6-7 and the dilution sealing film 6-8 are pierced, so that the respective chambers and the microfluidic pipe are in a connected state. Secondly, the sample control valve 6-10, the first PCR control valve 6-12, and the second PCR control valve 6-13 are closed, and the piston 6-4 is moved and pulled outward to a certain position, thereby the diluent in the dilution chamber 6-2 flowing to the injection chamber 6-3 through the dilution control valve 6-11. Thirdly, the dilution control valve 6-11 is closed, the sample control valve 6-10 is opened, and the piston 6-4 is moved back and forth so that the diluent in the injection chamber 6-3 enters the sample chamber 6-1 through the sample control valve 6-10. In the process of moving the piston 6-4 back and forth, the lysis freeze-dried powder in the sample chamber 6-1 is fully mixed with the diluent and the added sample evenly. Fourthly, the sample chamber 6-1 starts to be heated to a set temperature, so that the sample in the sample chamber 6-1 is fully lysed at the set temperature. Fifthly, after the lysis is completed, the piston 6-4 is moved and pulled outward to a certain position, so that the lysed sample mixture in the sample chamber 6-1 flows to the injection chamber 6-3 through the sample control valve 6-10. Sixthly, the sample control valve 6-10 and the dilution control valve 6-11 are closed, the first PCR control valve 6-12 and the second PCR control valve 6-13 are opened, and the piston 6-4 is moved back and forth so that the sample mixture in the injection chamber 6-3 enters the PCR chamber 6-5 through the first PCR control valve 6-12. During the back and forth movement of the piston 6-4, the lysed sample mixture is mixed evenly with the freeze-dried powder of the reverse transcriptase and the PCR raw materials of the PCR chamber 6-5. Ninthly, a PCR temperature increasing control for the PCR chamber 6-5 is started. In a prophase constant temperature stage of enzyme activation in PCR amplification, the mixture in the PCR chamber 6-5 will expand due to the high temperature, and the liquid overflowing in the expansion process can flow into the buffer chamber 6-6 through the second PCR control valve 6-13. After the constant temperature stage is over, the first PCR control valve 6-12 and the second PCR control valve 6-13 are closed, and the temperature cycle control of the PCR chamber 6-5 is started, and finally the amplification of the nucleic acid in the sample is completed.

According to an embodiment of the present disclosure, referring to FIGS. 9 to 11, the driving apparatus 300 includes a second motor 11, a third motor 13, thimbles 12, and a push rod 16. The second motor 11 is a linear motor, and the third motor 13 is a first screw motor. The second motor 11 is directly connected to the thimbles 12, and the third motor 13 is connected to the push rod 16 through a second nut 15. The second motor 11 and the thimbles 12 can be directly installed on the first base 1, and the third motor 13 can be installed on the first base 1 through a motor bracket 14. After the microfluidic kit containing the sample to be detected is fixed in the slot 6, the sample sealing film 6-7 and the dilution sealing film 6-8 in the above microfluidic kit respectively face one thimble 12, and then the thimbles 12 are driven to move linearly by the second motor 11, so that the sample sealing film 6-7 and the dilution sealing film 6-8 can be pierced, so as to connect the microfluidic pipe in the microfluidic kit. The third motor 13 can drive the push rod 16 to move linearly through the second nut 15 to control the back and forth movement of the piston 6-4 in the microfluidic kit, so that the sample in the kit flows in the microfluidic pipe of the kit..

It should be noted that protruding piercing members (not shown in the drawings) are provided inside the microfluidic pipe of the kit, and each piercing member has a tip portion. The piercing members are provided at positions opposed to the sample sealing film 6-7 and the dilution sealing film 6-8, and the tip portions of the piercing members face the sample sealing film 6-7 and the dilution sealing film 6-8. Thus, the thimbles 12 can press the piercing members to pierce the sample sealing film 6-7 and the dilution sealing film 6-8 without directly contacting the sample, and the microfluidic pipe is always isolated from the outside (device), thus effectively avoiding the contamination of the equipment by the sample. In addition, the PCR reactions in the kit are all carried out in a sealed PCR reaction chamber, and thus the problem of the the traditional PCR experiment in which the product is volatilized as aerosol into the device cavity will not occur.

According to an embodiment of the present disclosure, as shown in FIG. 11, the molecular detection system may further include: a linear guide rail 17 and a sliding block 18. The push rod 16 is arranged on the linear guide rail 17 through the sliding block 18. The push rod 16 and the sliding block 18 can be fixed by the second nut 15. Thus, under the driving of the third motor 13, the push rod 16 moves linearly on the linear guide rail 17, thereby controlling the expansion and contraction of the piston 6-4.

The detection apparatus 400 is adapted to detect the sample to be detected that has undergone the preprocessing, and the temperature control apparatus 500 is adapted to regulate the temperature of the sample to be detected. Specifically, the detection apparatus includes an optical path detection unit 19, and the temperature control apparatus 500 includes a chip temperature cycle control unit 7. While the temperature control apparatus 500 performs temperature cycle control of the sample in the PCR chamber 6-5, the detection apparatus 400 can perform real-time measurement of the amplified products in the PCR chamber 6-5 to obtain the fluorescence signal of the amplified products. Because probes are pre-arranged in the PCR chamber, the amplified products can hybridize with the probes and release fluorescent groups. As the amplified products increase, the fluorescent groups also increase continuously, which leads to the continuous increase of the fluorescence signal, which finally reaches the sensitivity range of the detection apparatus 400, causing a significant change in the detected data. Through analysis software, the number of cycles of the amplification when the significant change in this data occurs is defined as a CT value, and the detection results of the CT value appearing in a certain region are defined as positive, and the rest are defined as negative, so that the data of the fluorescence signal change is converted into a detection result of being negative or positive. According to a specific example of the present disclosure, the detection result can be obtained by PCR reaction of bacterial 16S rRNA. The primers used in the PCR reaction include forward primer 63f (5'-CAG GCC TAA CAC ATG CAA GTC-3') and reverse primer 1387r (5'-GGG CGG TGT GTA CAA GGC-3'). According to another specific example of the present disclosure, the detection result can be obtained by PCR reaction of influenza virus FluA. The primers used in the PCR reaction include the forward primer Forward (5'-CAGAGACTTGAAGATGTTTTTGC-3') and the reverse primer Reverse (5'-CTACGCTGCAGTCCTCGCTC-3'), and the probe used is Probe (5'-CAAGACCAATCCTGTCACCTCTGA-3 ').

According to an embodiment of the present disclosure, one end of the detection apparatus is connected to the slot through the temperature control apparatus. As shown in FIGS. 7 and 8, after the kit is fixed in the slot 6, the PCR chamber 6-5 in the kit is adjacent to the temperature cycle control unit 7 in the temperature control apparatus, so as to facilitate the temperature control apparatus to control the temperature of the PCR chamber 6-5.

According to an embodiment of the present disclosure, as shown in FIG. 2, the molecular detection system further includes a control system 20. The control system is respectively connected to the fixing apparatus, the driving apparatus, the detection apparatus and the temperature control apparatus, and is adapted for controlling the fixing apparatus, the driving apparatus, the detection apparatus and the temperature control apparatus to work collaboratively in order to detect the sample to be detected.

When addressing to solve the problem of mutual interference due to non-parallelism of the double guide rods in the linear sliding process, Applicant unexpectedly discovered that by sliding one of the double guide rods in a specific fixed sliding direction, and setting the other of the double guide rods to be movable in a specific sliding direction and in a direction perpendicular to the specific sliding direction, the two guide rods tend to be parallel to each other constantly during the sliding process, thereby eliminating the problem of mutual interference between the double guide rods in the linear sliding process.

### Sliding apparatus

According to an embodiment of the present disclosure, the molecular detection system of the present disclosure further includes a sliding apparatus connected to the driving apparatus and the fixing apparatus, and adapted to hold the container in a relatively fixed position. Referring to FIG. 13, the sliding apparatus includes a second base 13-10, a first guide rod 13-20, a second guide rod 13-30, a first connecting bracket 13-40, a second connecting bracket 13-50, and a first screw motor 13-60. One end of the second base 13-10 is provided with a fixed limit through hole 13-11, and the other end of the second base 13-10 is provided with a movable limit through hole 13-12; the first guide rod 13-20 is movably inserted in the fixed limit through hole 13-11; the second guide rod 13-30 is movably inserted in the movable limit through hole 13-12; the first connecting bracket 13-40 has a third through hole 13-41 provided therein, two ends of the first connecting bracket 13-40 are respectively connected to an upper end of the first guide rod 13-20 and an upper end of the second guide rod 13-30; two ends of the second connecting bracket 13-50 are respectively connected to a lower end of the first guide rod 13-20 and a lower end of the second guide rod 13-30; the first screw motor 13-60 is arranged on the second base 13-10 and a screw 13-61 on the first screw motor 13-60 is perpendicular to the second base 13-10, and the screw 13-61 extends through the third through hole 13-41 and drives the first connecting bracket 13-40 to move and jointly moves the first guide rod 13-20, the second guide rod 13-30 and the second connecting bracket 13-50. When the screw 13-61 of the first screw motor 13-60 rotates, it will drive the first connecting bracket 13-40 to move with the screw 13-61, and at the same time, the first bracket 13-40 drives the first guide rod 13-20, the second guide rod 13-30 and the second connecting bracket 13-50 to move. At this time, the first guide rod 13-20 slides along the fixed limit through hole 13-11 when moving, and the fixed limit through hole 13-11 makes the first guide rod 13-20 slide along a longitudinal direction of the second base 13-10 and in a basically unchanged sliding direction, while the second guide rod 13-30 slides in the movable limit through hole 13-12, and the movable limit through hole 13-12 can provide a certain movement space for the second guide rod 13-30 in a transverse direction of the second base 13-10, so that the space for the passing of the second guide rod 13-30 in the second base 13-10 increases as the second guide rod 13-30 moves with the first bracket 13-40. Therefore, even if the first guide rod 13-20 and the second guide rod 13-30 cannot be guaranteed to be parallel in the installation process, on the basis that the movement direction of the first guide rod 13-20 is basically unchanged and by means of the movable limit through hole 13-12, the second guide rod 13-30 can slide smoothly in the through hole of the second base without changing its original fixed direction, thereby effectively protecting the guide rods from being interfered by the second base 13-10 during the movement. In summary, the sliding apparatus is not only simple in structure and the guide rods are easy to install, and the double guide rods can slide smoothly in the through holes of the second base 13-10 when the double guide rods are not parallel. It should be noted that the "transverse direction" in the present disclosure is as shown in FIG. 13, the "longitudinal direction" is a direction perpendicular to the "transverse direction", and the "upper end" and the lower end are based on the direction from bottom to top in FIG. 13.

According to a specific embodiment of the present disclosure, the inner diameter of the fixed limit through hole 13-11 is larger than the diameter of the first guide rod 13-20 by 0.1mm to 0.3mm. Applicant found that if the difference between the inner diameter of the fixed limit through hole 13-11 and the diameter of the first guide rod 13-20 is too large, the first guide rod 13-20 may also slide along the transverse direction of the second base 13-10 when sliding along the longitudinal direction of the second base 13-10, which not only fails to effectively fix the sliding direction of the first guide rod 13-20, but also causes the overall sliding direction of the first connecting bracket 13-40, the second connecting bracket 13-50 and the second guide rod 13-20 to constantly change, thereby causing the disordered movement of the guide rods on the second base 13-10, which greatly reduces the smoothness of the guide rods sliding on the second base 13-10; if the difference between the inner diameter of the fixed limit through hole 13-11 and the diameter of the first guide rod 13-20 is too small, it will increase the difficulty of the first guide rod 13-20 sliding along the longitudinal direction of the second base 13-10. In the present disclosure, the inner diameter of the fixed limit through hole 13-11 is controlled to be 0.1mm to 0.3mm larger than the diameter of the first guide rod 13-20, which not only enables the first guide rod 13-20 to slide smoothly in the second base 13-10, but also ensures that the sliding direction of the first guide rod 13-20 is basically unchanged during the sliding process.

According to another specific embodiment of the present disclosure, an inner diameter of the movable limit through hole 13-12 is 0.5mm to 2mm larger than a diameter of the second guide rod 13-30. In fact, in the sliding apparatus of the above embodiment of the present disclosure, after the double guide rods and the connecting brackets are fixed, even when the first guide rod 13-20 and the second guide rod 13-30 are not parallel, the direction of movement of the second guide rod 13-30 during the sliding process of the two rods actually remains basically unchanged, but because the first guide rod 13-20 and the second guide rod 13-30 are not parallel, the distance between them in the transverse direction of the second base 13-10 changes continuously when they pass through the second base 13-10. Applicant found that if the difference between the inner diameter of the movable limit through hole 13-12 and the diameter of the second guide rod 13-30 is too small, it is not enough to eliminate the interference of the second base 13-10 with the movement of the guide rod; if the difference between the inner diameter of the movable limit through hole 13-12 and the diameter of the second guide rod 13-30 is too large, the smooth operation of the guide rod cannot be ensured. In the present disclosure, the inner diameter of the movable limit through hole 13-12 is controlled to be larger than the diameter of the second guide rod by 0.5mm to 2mm, which can not only eliminate the interference problem caused by the double guide rods being not parallel, making the double guide rods slide smoothly in the through holes of the second base 13-10 when they are not parallel, but also ensure that the guide rods move smoothly.

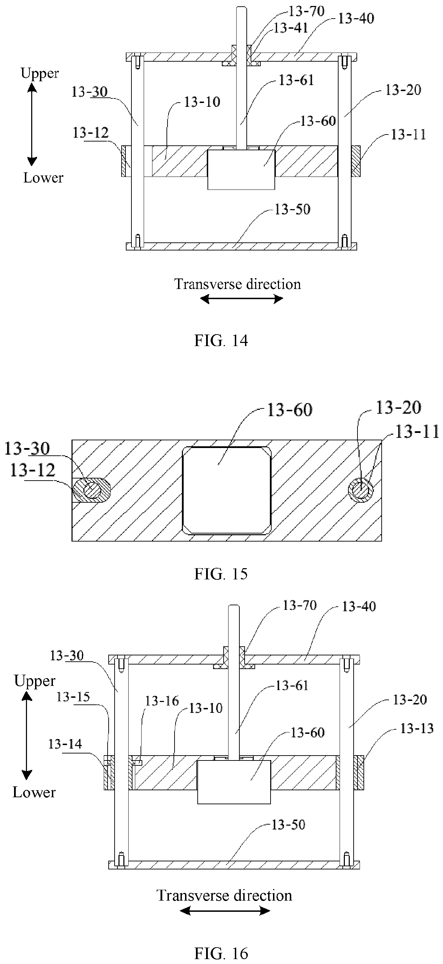

According to another specific embodiment of the present disclosure, the cross section of the movable limiting through hole 13-12 along the longitudinal direction of the second base 13-10 may be circular, oval or capsule-like. The inner diameter of the movable limiting through hole 13-12 is greater than the diameter of the second guide rod 13-30 by 0.5mm to 2mm, so that sufficient movement space for the second guide rod 13-30 can be provided in the transverse direction of the second base 13-10. When the cross section of the movable limit through hole 13-12 in the longitudinal direction of the second base 13-10 has an ellipse or capsule shape, the long axis of the ellipse can be parallel to the direction from the movable limit through hole 13-12 to the fixed limit through hole 13-11, the length of the capsule shape (e.g., shown in FIG. 15) can be parallel to the direction from the movable limit through hole 13-12 to the fixed limit through hole 13-11, and the width of the capsule shape can be the same as the inner diameter of the fixed limit through hole 13-11, which not only eliminates the interference problem caused by the double guide rods being not parallel, making the double guide rods slide smoothly in the through holes of the second base 13-10 when they are not parallel, but also further ensures the smooth movement of the guide rods.

According to another specific embodiment of the present disclosure, as shown in FIG. 16, the second base 13-10 may further include a fixed shaft sleeve 13-13 and a movable shaft sleeve 13-14. The fixed shaft sleeve 13-13 is fixed in the fixed limit through hole 13-11. The first guide rod 13-20 slides through the fixed shaft sleeve 13-13; the movable shaft sleeve 13-14 is fixed in the movable limit through hole 13-12, and the movable shaft sleeve 13-14 is provided with a sliding member 13-15 outside. A sliding track 13-16 that matches the sliding member 13-15 is provided within the second base 13-10. The sliding track 13-16 allows the movable shaft sleeve 13-14 to move along the longitudinal and transverse directions of the second base 13-10. Therefore, it is possible to further ensure the smooth movement of the guide rods on the basis of ensuring that the double guide rods can slide smoothly in the second base 13-10 under the condition that they are not parallel.

According to another specific embodiment of the present disclosure, two ends of the first connecting bracket 13-40 are threadedly connected to an upper end of the first guide rod 13-20 and an upper end of the second guide rod 13-30 through bolts, respectively, and two ends of the second connecting bracket 13-50 are threadedly connected to a lower end of the first guide rod 13-20 and a lower end of the second guide rod 13-30 through bolts, respectively.

According to another specific embodiment of the present disclosure, the manner for fixing the screw 13-61 with the third through hole 13-41 is not particularly limited, and those skilled in the art can make selections according to actual needs. For example, the screw 13-61 is connected to the third through hole 13-41 through screw threads. Therefore, there is no need to set a fixing nut, and the direct threaded connection between the screw 13-61 and the third through hole 13-41 can make the screw 13-61 and the first connecting bracket 13-40 relatively fixed, so that the rotation of the screw 13-61 can drive the first connecting bracket 13-40 to move in the direction of the screw, and jointly move the first guide rod 13-20, the second guide rod 13-30 and the second connecting bracket 13-50.

According to another specific embodiment of the present disclosure, referring to FIG. 14, the above sliding apparatus may further include: a first screw nut 13-70 matching and connected to the screw 13-61 and fixedly connected to the third through hole 13-41. Therefore, the fixing strength of the screw 13-61 and the first connecting bracket 13-40 can be further improved, thereby effectively preventing the problem of abnormal movement due to the loose connection between the first connecting bracket 13-40 and the screw 13-61.

According to another specific embodiment of the present disclosure, the sliding apparatus described above is applied to the molecular detection system to automatically control the pressing and fixing or loosening of the kit. At this time, the first connecting bracket 13-40 is movable within an interval of 5mm to 55mm from the second base 13-10, which is convenient for the fixation or replacement of the kit, improving the detection efficiency.

According to another specific embodiment of the present disclosure, the types of the motor and the screw 13-51 in the first screw motor 13-50 of the present disclosure are generally not limited, and those skilled in the art can make selections according to actual needs. For example, the motor may be a direct current motor, a stepper motor or a servo motor, and the screw 13-51 may be a trapezoidal screw, a ball screw or a planetary ball screw. Therefore, the applicability of the present disclosure can be further improved.

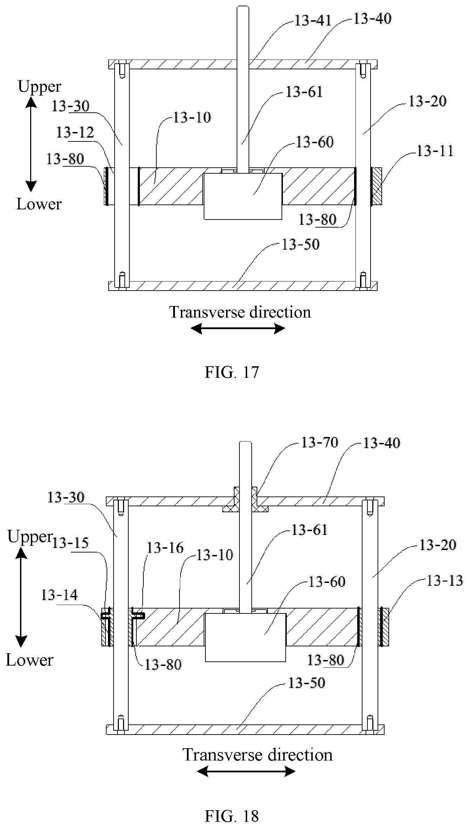

According to another specific embodiment of the present disclosure, as shown in FIGS. 17 and 18, a lubricating layer 13-80 is formed on a surface of each of the fixed limit through hole 13-11 and the movable limit through hole 13-12, which can further reduce the friction and wear that may be generated between the first guide rod and the second guide rod and the second base during the sliding process, thereby further reducing the interference of the second base with the guide rods. It should be noted that the material of the lubricating layer 13-80 in the present disclosure is not particularly limited, and those skilled in the art can selections according to actual needs. For example, the lubricating layer 80 may be formed of a liquid lubricant, a semisolid lubricant or a solid lubricant, thereby effectively reducing the friction and wear that may occur between the double guide rods and the second base 13-10.

According to another specific embodiment of the present disclosure, the lubricating layer 13-80 may be a lubricating oil layer or a graphite layer, thereby not only ensuring that the lubricating layer 80 has better lubricating performance at room temperature, but also preventing the limit through holes from rusting, thereby further avoiding the interference of the second base during the movement of the guide rods. Further, the thickness of the lubricating layer 13-80 may be 0.01mm to 1mm, for example, 0.05mm to 0.5mm, 0.1mm, 0.2mm, 0.3mm, 0.4mm, 0.5mm, 0.6mm, 0.7mm, 0.8mm, or 0.9mm, etc. Therefore, the interference of the second base to the guide rods during the movement can be further avoided.

In addition, in another aspect of the present disclosure, the present disclosure also proposes a sliding apparatus. Those skilled in the art can understand that the sliding apparatus has all the features and advantages of the sliding apparatus described above, and will not be repeated here.

### Optical path imaging automatic adjustment apparatus

According to an embodiment of the present disclosure, the molecular detection system of the present disclosure may further include: an optical path imaging automatic adjustment apparatus connected to the detection apparatus and including a photoelectric sensor and a convex lens, and the optical path imaging automatic adjustment apparatus is adapted to automatically adjust a distance between the photoelectric sensor and the convex lens. In the optical path imaging automatic adjustment apparatus, rotation of the screw of the second screw motor will drive the adjusting bracket to move with the screw, and the convex lens is arranged on the adjusting bracket so that the movement of the adjusting bracket will also drive the convex lens to move, that is, the convex lens moves back and forth along the direction of the screw, thereby changing the relative distance from the convex lens to the photoelectric sensor, and then changing the imaging position of the convex lens. Finally, the imaging through the convex lens just falls in a receiving zone of the photoelectric sensor, realizing the automatic adjustment of imaging. As a result, the optical path imaging automatic adjustment apparatus can not only realize the detection of objects to be detected (i.e., to-be-detected samples) at different object distances in the optical path system, but also obtain the best and clearest image.

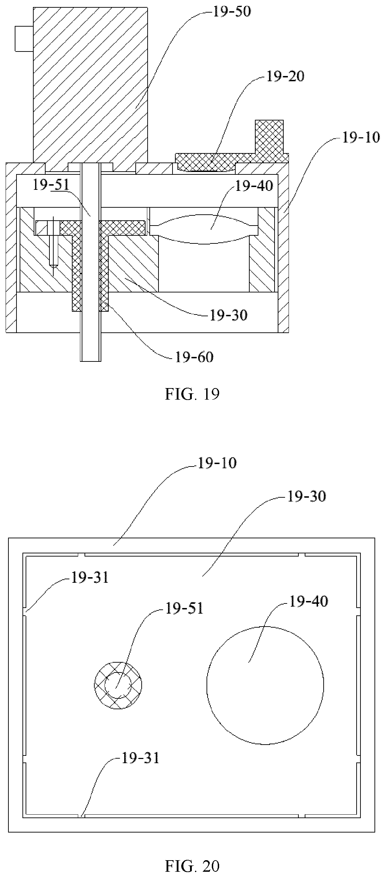

According to an embodiment of the present disclosure, as shown in FIG. 19, the above optical path imaging automatic adjustment apparatus includes a third base 19-10, a photoelectric sensor 19-20, an adjusting bracket 19-30, a convex lens 19-40, and a second screw motor 19-50. The photoelectric sensor 19-20 is fixed on the third base 19-10, and the adjusting bracket 19-30 is movably arranged on the third base 19-10. The convex lens 19-40 is provided on the adjusting bracket 19-30, and the distance between the convex lens 19-40 and the photoelectric sensor 19-20 can be adjusted by moving the adjusting bracket 19-30. The second screw motor 19-50 is fixed on the third base 19-10 and the screw 19-51 on the second screw motor 19-50 passes through the adjusting bracket 19-30 and drives the adjusting bracket 19-30 to move. In the optical path imaging automatic adjustment apparatus, rotation of the screw 19-51 of the second screw motor 19-50 will drive the adjusting bracket 19-30 to move with the screw 19-51, and because the convex lens 19-40 is provided on the adjusting bracket 19-30 the movement of the adjusting bracket 19-30 will drive the convex lens 19-40 to move, that is, the convex lens 19-40 moves back and forth along the direction of the screw 19-51, thereby changing the relative distance from the convex lens 19-40 to the photoelectric sensor 19-20 and then changing the imaging position of the convex lens 19-40. Finally, the imaging through the convex lens 19-40 just falls in the receiving zone of the photoelectric sensor 19-20, realizing the automatic adjustment of imaging. As a result, the optical path imaging automatic adjustment apparatus can not only realize the detection of objects to be detected (i.e., to-be-detected samples) at different object distances in the optical path system, but also obtain the best and clearest image.

According to a specific embodiment of the present disclosure, the manner for fixing the screw 19-51 with the adjusting bracket 19-30 in the present disclosure is not particularly limited, and those skilled in the art can make selections according to actual needs. For example, the screw 19-51 is connected to the adjusting bracket 19-30 by screw threads, so that the screw 19-51 and the adjusting bracket 19-30 can be relatively fixed, and the fixing strength of the two can be improved, so that rotation of the screw 19-51 can drive the adjusting bracket to move along the direction of the screw 19-51, and change the relative distance from the convex lens 19-40 to the photoelectric sensor 19-20.

According to another specific embodiment of the present disclosure, the optical path imaging automatic adjustment apparatus may further include: a second screw nut 19-60 matching and connected to the screw 19-51 and fixedly connected to the adjusting bracket 19-30. Therefore, the fixing strength of the screw 19-51 and the adjusting bracket 19-30 can be further improved, and the problem of abnormal movement due to the loose connection between the adjusting bracket 19-30 and the screw 19-51 can be effectively prevented.

According to another specific embodiment of the present disclosure, the optical path imaging automatic adjustment apparatus described in the present disclosure can be applied to a molecule automatic detection system to obtain the best and clearest image. In this case, the maximum movable distance of the adjusting bracket 19-30 is 30mm. For example, the moving distance of the adjusting bracket 19-30 may be 5mm, 10mm, 15mm, 20mm, or 25mm, etc. Therefore, the distance between the convex lens 19-40 and the photoelectric sensor 19-20 can be controlled by the adjusting bracket 19-30, and the distance between the two can be changed within the range of 30mm to 60mm, which can further help to obtain the best and clearest image.

According to another specific embodiment of the present disclosure, as shown in FIG. 20, a plurality of raised ribs 19-31 are formed on a peripheral wall of the adjusting bracket 19-30, and the raised ribs 19-31 abut against an inner wall of the third base 19-10. In the present disclosure, by adopting the above settings, the contact area between the adjusting bracket 19-30 and the third base 19-10 can be effectively reduced, thereby reducing the friction generated during mutual movement of the adjusting bracket 19-30 and the third base 19-10. According to another specific embodiment of the present disclosure, the photoelectric sensor 19-20 and the second screw motor 19-50 are intelligently and jointly controlled through a CPU end. By adopting the above settings of the present disclosure, the activation or inactivation of the second screw motor can be controlled according to the definition of the obtained image, so as to obtain the best and clearest image. Specifically, when the obtained image is not clear, the CPU terminal controls the second screw motor 19-50 to be activated, and constantly adjusts the distance between the convex lens 19-40 and the photoelectric sensor 19-20 through the rotation of the screw 19-51 until the clearest image is obtained. When the obtained image definition reaches the requirement, the CPU terminal controls the second screw motor 19-50 to be inactivated. As a result, it is possible to further realize the detection of objects to be detected at different object distances in the optical path system to obtain the best and clearest image.

According to another specific embodiment of the present disclosure, the types of the motor and the screw 19-51 in the second screw motor 19-50 of the present disclosure are generally not limited, and those skilled in the art can make selections according to actual needs. For example, the motor may be a direct current motor, a stepper motor or a servo motor, and the screw 19-51 may be a trapezoidal screw, a ball screw, or a planetary ball screw. Therefore, the applicability of the present disclosure can be further improved.

In addition, in another aspect of the present disclosure, the present disclosure also provides an optical path imaging automatic adjustment apparatus. Those skilled in the art can understand that the optical path imaging automatic adjustment apparatus has all the features and advantages described above for the optical path imaging automatic adjustment apparatus, and will not be repeated here.

### Slot

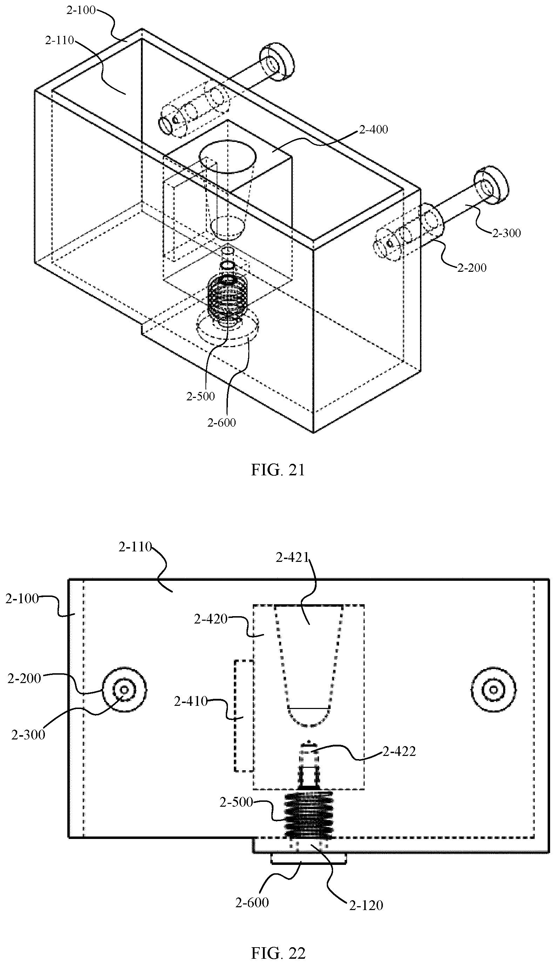

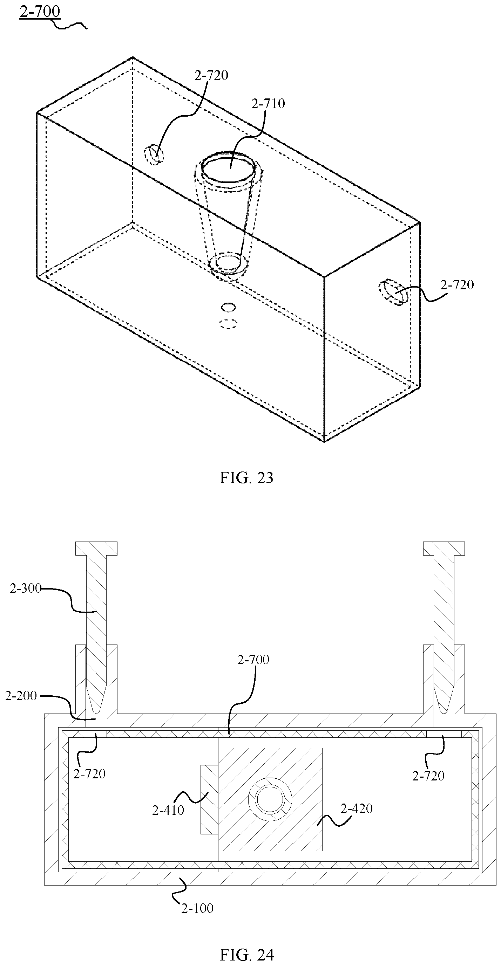

According to an embodiment of the present disclosure, referring to FIGS. 21 and 23, the slot in the molecular detection system proposed by the present disclosure includes: a housing 2-100, at least one positioning hole 2-200, at least one positioning pin 2-300, a heating assembly 2-400, and a second compression spring 2-500. The upper part of the housing 2-100 is open and defines an accommodating space 2-110 inside; the at least one positioning hole 2-200 is provided in a side portion of the housing 2-100; one end of each of the at least one positioning pin 2-300 extends into one of the at least one positioning hole 2-200; the heating assembly 2-400 is arranged in the accommodating space 2-110; and the second compression spring 2-500 is arranged at a bottom of the accommodating space 2-110 and is connected to the heating assembly 2-400.

By adopting the above slot, after the kit is put into the accommodating space of the housing, a part to be heated of the kit can naturally fall on the heating assembly, and an opening in the kit for positioning (pre-processed positioning opening) is located slightly higher than the position of the positioning hole in the slot for the kit. By moving the positioning pin along the positioning hole toward the kit, the kit will move slightly downward as the positioning pin is locked into the positioning opening, and the second compression spring will be compressed and deformed. Under the fit action of the positioning pin and the second compression spring, the part to be heated of the kit can be closely attached to the heating assembly in the slot. Therefore, by using the slot of the present disclosure to fix and heat the kit, the processing of the kit only needs to meet the requirements that the part to be heated matches the heating assembly, and the positioning opening matches the positioning pin, thereby greatly reducing the processing cost of the kit, Besides, because the part to be heated of the kit is tightly and stably attached to the heating assembly, the heating requirements of the kit in the experiment can be satisfied.

According to the embodiment of the present disclosure, referring to FIGS. 21 and 22, the bottom of the housing 2-100 is further provided with a via hole 2-120, the slot further includes a fixing screw 2-600 extending through the via hole 2-120 into the accommodating space 2-110 to be connected to the heating assembly 2-400, and the second compression spring 2-500 is sleeved on the fixing screw 2-600. By mounting the heating assembly 2-400 and the second compression spring 2-500 on the fixing screw 2-600, it can further facilitate the installation of the heating assembly 2-400 and the second compression spring 2-500 in the accommodation space 2-110 of the housing, and further improve the stability of the installation of the heating assembly 2-400 and the second compression spring 2-500. After the kit is placed in the accommodating space 2-110, the part to be heated thereof can naturally fall on the heating assembly 2-400, and slightly presses the second compression spring 2-500 downward on the fixing screw 2-600.

According to the embodiment of the present disclosure, the at least one positioning hole 2-200 includes a plurality of of positioning holes 2-200, the at least one positioning pin 2-300 includes a plurality of positioning pins 2-300, and the plurality of positioning holes 2-200 is arranged in one-to-one correspondence with the plurality of positioning pins 2-300, that is, each positioning hole 2-200 has one positioning pin 2-300 inserted therein. By arranging the plurality of positioning holes 2-200 and the plurality of positioning pins 2-300, on the one hand, it further facilitates to press down the kit to be fixed by using the positioning pins 2-300, so that the part to be heated is closely attached to the heating assembly 2-400; on the other hand, it can further improve the stability of the kit after being fixed, and avoid displacement of the kit in the experiment. According to a specific example of the present disclosure, as shown in FIGS. 21, 22, and 24, two sets of positioning holes 2-200 and positioning pins 2-300 can be provided on two sides of one side wall of the housing 2-100, respectively. Therefore, it further facilitates to press down the kit to be fixed by using the positioning pins 2-300, enable the part to be heated to be closely attached to the heating assembly 2-400, and further improve the stability of the kit after being fixed.

According to the embodiment of the present disclosure, referring to FIGS. 21 and 24, the end of the positioning pin 2-300 extending into the positioning hole 2-200 is conical, in other words, along the direction in which the positioning pin 2-300 extends into the positioning hole 2-200, the cross-sectional area of the positioning pin 2-300 gradually decreases, and the head of the positioning pin 2-300 changes in a cone angle. By setting the end of the positioning pin 2-300 extending into the positioning hole 2-200 to be conical, it is more convenient for the positioning pin 2-300 to press the kit downward when being locked into the corresponding positioning opening of the kit.

According to an embodiment of the present disclosure, the heating assembly 2-400 includes a heating pad 2-410 and a heat conduction block 2-420 that are connected to each other, and the heat conduction block 2-420 is connected to the second compression spring 2-500. According to a specific example of the present disclosure, as shown in FIGS. 21, 22, 24, and 25, the heating pad 2-410 may be provided on one side of the heat conduction block 2-420. In operation, the heating pad 2-410 can be controlled to generate heat, and the heating pad 2-410 can be used to indirectly heat the heat conduction block 2-420. As a result, the temperature of the entire heat conduction block 2-420 is relatively uniform, which can further improve the heating effect on the part to be heated in the kit.

According to an embodiment of the present disclosure, as shown in FIG. 22, a heating chamber 2-421 can be formed on the heat conduction block 2-420. It should be noted that the specific shape of the heating chamber 2-421 is not particularly limited. The heating chamber 2-421 can be processed into a shape that matches the shape of the part to be heated in the kit, so that the part to be heated in the microfluidic kit can be closely attached to the heating chamber 2-421.

According to the embodiment of the present disclosure, as shown in FIG. 22, a lower part of the heat conduction block 2-420 is provided with a limiting hole 2-422, and the fixing screw 2-600 can extend into the limiting hole 2-422, which is convenient for the connection between the heat conduction block and the fixing screw 2-600. According to a specific example of the present disclosure, the fixing screw 2-600 and the heat conduction block 2-420 are threadedly connected through the limiting hole 2-422.

According to the embodiment of the present disclosure, the hole diameter of the via hole 2-120 is larger than the diameter of the fixing screw 2-600. As a result, it is easier for the fixing screw 2-600 to pass through the via hole 2-120 and the fixing screw 2-600 can move in arbitrary direction within a certain range of the housing 2-100, and the heating module 2-400 can move within a certain range with the fixing screw 2-600, and is also subjected to the pressure generated by the deformation of the second compression spring 2-500, so as to be closely attached to the part to be heated of the kit.

In order to facilitate understanding, the installation and cooperation relationship between the slot and the kit of the present disclosure will be described in detail below with reference to FIGS. 23-25.

The structure of the kit adapted for the slot of the present disclosure is shown in FIG. 23, where the kit 2-700 includes a to-be-heated part 2-710 and positioning openings 2-720. By adopting the slot of the present disclosure, the kit can be stably fixed to the slot through the positioning openings 2-720 processed on the kit. According to a specific example of the present disclosure, the kit 2-700 may include two positioning openings 2-720, wherein one positioning opening 2-720 is a circular hole, and the other positioning opening 2-720 is a waist-shaped hole. As a result, the degree of freedom that the kit 2-700 can move in the slot can be further expanded, thereby further ensuring the stability of the microfluidic kit 2-700 being fixed by the positioning pin 2-300 and the second compression spring 2-500, and allowing the to-be-heated part 2-710 of the kit 2-700 to be closely attached to the heating chamber 2-421 of the heat conduction block 2-420.

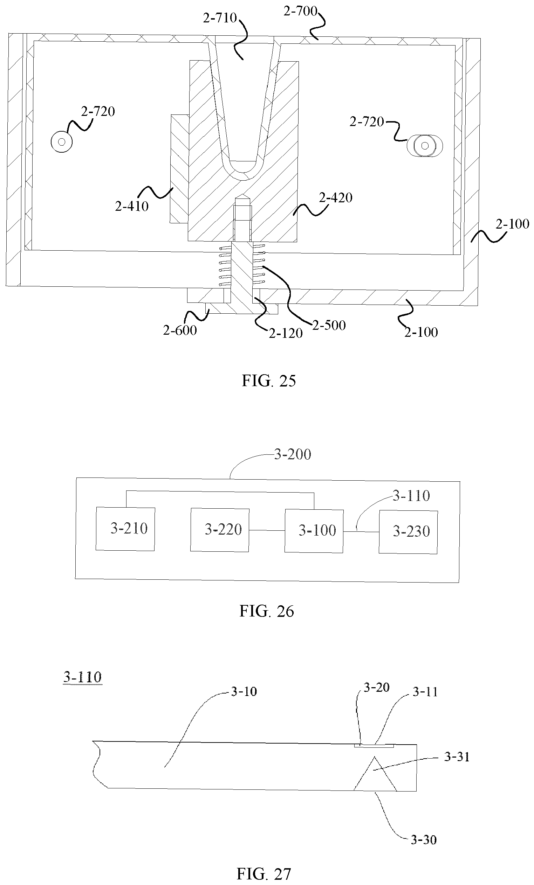

Referring to FIG. 24, after the kit 2-700 is inserted from the top of the slot, the to-be-heated part 2-710 enters the heating chamber 2-421, and the two positioning openings 2-720 face the positioning holes 2-200 and the positioning pins 2-300, respectively. By controlling the positioning pins 2-300 to move along the positioning holes 2-200 towards the the kit 2-700, the positioning pins 2-300 are locked into the positioning openings 2-720. During the process of the positioning pins 2-300 being locked into the positioning openings 2-720, the conical heads of the positioning pins 2-300 drive the kit 2-700 to move downward through the positioning openings 2-720, and cause the second compression spring 2-500 to be further compressed. At the same time, the fixing screw 2-600 can also drive the heat conduction block 2-420 to move freely within a certain range. After the fixation is completed, as shown in FIG. 25, under the cooperative action of the positioning pins 2-300 and the second compression spring 2-500, the to-be-heated part of the kit 2-700 can be closely attached to the heating chamber 2-421 of the heat conduction block 2-420.

In summary, the molecular detection system proposed by the present disclosure uses the slot of the above embodiment to fix and heat the kit, which has low processing requirements for the kit, and the to-be-heated part of the kit is closely and stably attached to the heating assembly, satisfying the heating requirements of the kit in the experiment.

In addition, in another aspect of the present disclosure, the present disclosure also proposes a slot. Those skilled in the art can understand that the slot has all the features and advantages described above for the slot, and will not be repeated here.

In another aspect of the present disclosure, the present disclosure provides a microfluidic kit adapted for the molecular detection system of the above embodiment. According to an embodiment of the present disclosure, as shown in FIGS. 26 and 27, the microfluidic kit includes: a box body 3-200 and a microfluidic kit chip 3-100. The box body 3-200 has a reagent zone 3-210, a sample zone 3-220, and a reaction zone 3-230; the microfluidic kit chip 3-100 includes a microfluidic pipe 3-110 with a piercing member 3-30 provided inside and facing a liquid outlet 3-11, and the microfluidic kit chip 3-100 is connected respectively to the reagent zone 3-210, the sample zone 3-220 and the reaction zone 3-230 through the microfluidic pipe 3-110. The microfluidic kit can not only introduce the sample to be detected in the sample zone and the reagent in the reagent zone (if the reaction zone has a freeze-dried powder reagent, the reagent zone can also store a diluent) into the reaction zone for reaction through the microfluidic pipe, so as to realize the detection of the sample, and there is no need to set extra external parts such as an elastic valve to realize the disconnection and connection of the pipe.

According to an embodiment of the present disclosure, as shown in FIG. 27, the microfluidic pipe 3-110 used for the microfluidic kit chip 3-100 includes a pipe 3-10, a sealing film 3-20, and a piercing member 3-30. An end portion of the pipe 3-10 is made of an elastic material, and the liquid outlet 3-11 is provided in a side wall of the end portion of the pipe 3-10; the sealing film 3-20 is arranged at the liquid outlet 3-11 to seal the liquid outlet 3-11. The piercing member 3-30 has a tip portion 3-31, the piercing member 3-30 is provided on the inner wall of the end portion of the pipe 3-10 opposed to the liquid outlet 3-11, and the tip portion 3-31 faces the liquid outlet 3-11 and is configured to pierce through the sealing film 3-20 when the end portion is squeezed under an external force. The microfluidic pipe is sealed by the sealing film 3-20 provided at the liquid outlet 3-11 of the pipe 3-10, the piercing member 3-30 with the tip portion 3-31 is provided on the inner wall of the end portion of the pipe 3-10 opposed to the liquid outlet 3-11, and the tip portion 3-31 faces the liquid outlet 3-11, so that the microfluidic pipe can be in a sealed state before use, and a force can be applied to the end portion of the pipe 3-10 in advance when in use to allow the piercing member 3-30 to pierce the sealing film. Therefore, the sealing of the microfluidic pipe can be realized without external pressure parts when storing raw materials in the pipe, which simplifies the operation steps when the microfluidic pipe is used. It should be noted that the end portion of the pipe 3-10 in the present disclosure refers to the end of the microfluidic pipe adjacent to the liquid outlet. In addition, the piercing member is also called a penetrating member, and the liquid outlet is also called a liquid exit opening.

According to a specific embodiment of the present disclosure, the piercing member 3-30 and the liquid outlet 3-11 may be located on the same pipe diameter of the pipe 3-10. As a result, the piercing member 3-30 can be located to just face the sealing film 3-20 at the liquid outlet, and when a force is applied to the end portion of the pipe 3-10, it is more favorable for the tip portion 3-31 of the piercing member to pierce the sealing film 3-20.

According to another specific embodiment of the present disclosure, in the present disclosure, the distance between the tip portion 3-31 of the piercing member and the sealing film 3-20 is not particularly limited, and those skilled in the art can make selections according to actual specifications and needs of the microfluidic pipe. For example, the distance between the tip portion 3-31 of the piercing member and the sealing film 3-20 may be 0.05mm to 4mm, 0.05mm to 2mm, or 0.05mm to 1mm, etc. As a result, it can not only ensure that the piercing member 3-30 will not pierce the sealing film 3-20 when the end of the pipe 3-10 is not subjected to external force, that is, ensure that the microfluidic pipe is in a sealed state during the storage of raw materials, but also ensure that the sealing film 3-20 can be pierced through when the force is applied to the end portion of the pipe 3-10, so that the raw materials stored in the microfluidic pipe can seep out from the liquid outlet 3-11.

According to another specific embodiment of the present disclosure, the sealing film 3-20 may be a film material that does not have a permeation effect, such as a tin foil paper, a laminating film or a kraft paper. This can effectively prevent the raw materials stored in the microfluidic pipe from seeping out through permeation when the pipe 3-10 is in a sealed state.

According to another specific embodiment of the present disclosure, the thickness of the sealing film 3-20 may be 0.01mm to 0.2mm. Applicant found that if the thickness of the sealing film 3-20 is too small, the sealing film is easily damaged, which increases the difficulty in sealing the liquid outlet 3-11 with the sealing film 3-20. If the thickness of the sealing film 3-20 is too large, it will increase the difficulty of piercing the sealing film 3-20. In the present disclosure, by controlling the thickness of the sealing film 3-20 to be 0.01mm to 0.2mm, it is not only beneficial to seal the liquid outlet 3-11, but also reduces the difficulty in piercing the sealing film 3-20 by the piercing member 3-30.

According to another specific embodiment of the present disclosure, as shown in FIGS. 27-29, the tip of the tip portion 3-31 of the piercing member 3-30 may have a tapered structure, such as a cone or a polygonal pyramid, so that the external force required for the piercing member 3-30 to pierce the sealing film 3-20 can be significantly reduced, being conducive to the piercing of the sealing film 3-20.

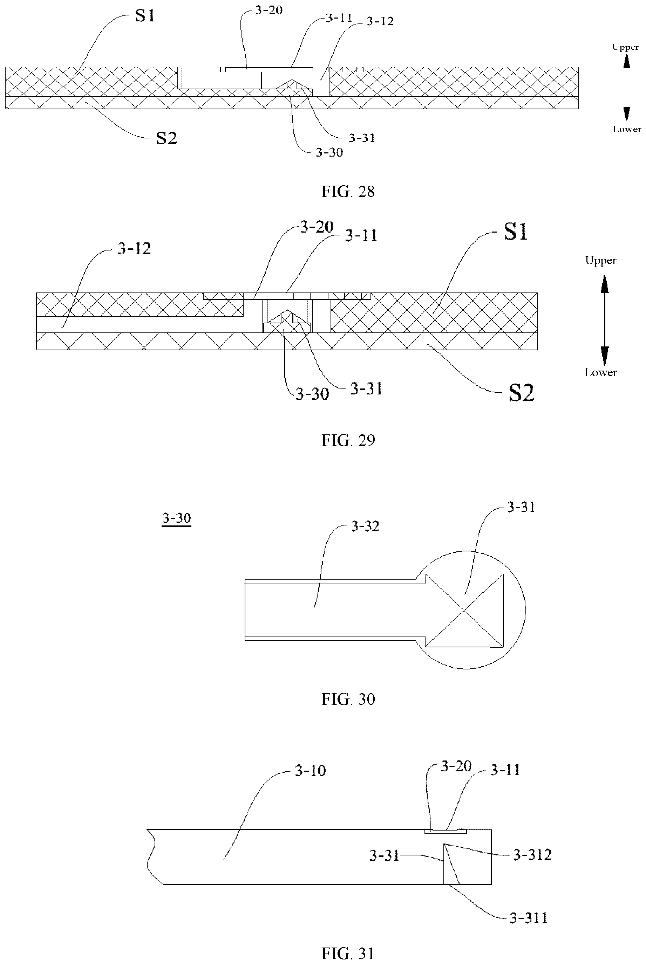

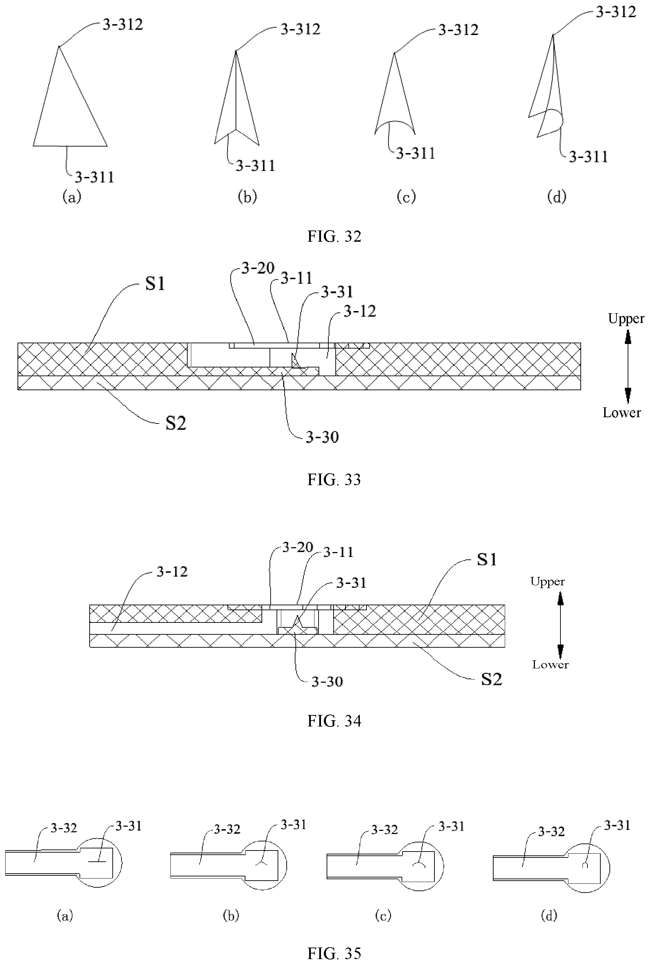

According to another specific embodiment of the present disclosure, as shown in FIGS. 31 to 35, the tip portion 3-31 of the piercing member 3-30 may be formed by a triangular surface, and one side 3-311 of the triangular surface is provided on the inner wall of the end portion of the pipe opposed to the liquid outlet 3-11, and the apex angle 3-312 corresponding to the side 3-311 of the triangular surface forms the tip of the tip portion 3-31, which can significantly reduce the external force required for the piercing member 3-30 to pierce the sealing film 3-20, and further facilitates the piercing of the sealing film 3-20. Further, the shape of the side 3-311 of the triangular face provided on the inner wall of the end portion of the pipe determines the shape of the tip portion 3-31. As shown in FIG. 35 (a), FIG. 35 (b), FIG. 35 (c), and FIG. 35(d), the side 3-311 may be straight line-shaped, V-shaped, arc-shaped or U-shaped, and thus the tip portion 3-31 can be a plat surface, two intersecting flat surfaces, an arc surface or a U-shape curved surface (as shown in FIG. 32 (a), FIG. 32 (b), FIG. 32 (c), FIG. 32 (d) in sequence), which can further improve the stability of the tip portion 3-31. In addition, the angle of the apex angle 3-312 of the tip of the triangular surface forming the tip portion 3-31 may be smaller than or equal to 120 degrees, for example, 10 degrees, 30 degrees, 60 degrees, 70 degrees, 80 degrees, 90 degrees, 100 degrees or 110 degrees. Adopting this design can make the tip of the tip portion 3-31 have a smaller volume, which can further reduce the external force required for the piercing member 3-30 to pierce the sealing film 3-20, thereby being further conducive to piercing the sealing film. Preferably, the angle of the apex angle 3-312 of the tip of the triangular surface 3-310 forming the tip portion 3-31 may be smaller than or equal to 90 degrees, for example, 5 degrees, 10 degrees, 20 degrees, 25 degrees, 40 degrees, 50 degrees, 65 degrees, 75 degrees, 85 degrees, or 90 degrees, so that the volume of the tip of the tip portion 3-31 can be further reduced, thereby further facilitating the piercing of the sealing film.

According to another specific embodiment of the present disclosure, the thickness of the triangular surface may be smaller than or equal to 1mm, for example, 0.1mm, 0.2mm, 0.3mm, 0.4mm, 0.5mm, 0.6mm, 0.7mm, 0.8mm or 0.9mm, etc. Therefore, the contact area between the tip portion 3-31 and the sealing film 3-20 can be further reduced, and the external force required for the piercing member 3-30 to pierce the sealing film 3-20 can be further reduced, thereby further facilitating the piercing of the sealing film 3-20. Preferably, along a direction from the tip portion 3-31 toward the liquid outlet 3-11, the thickness of the triangular surface 3-310 gradually decreases, so that the volume of the tip portion 3-31 can be further reduced on the basis of ensuring that the tip portion 3-31 has better stability, thereby being further conducive to piercing the sealing film.

According to another specific embodiment of the present disclosure, as shown in FIG. 28, FIG. 29, FIG. 33 and FIG. 34, the piercing member 3-30 and the pipe 3-10 may be integrally formed into one piece. This not only further simplifies the structure of the microfluidic pipe, but also fixes the relative positions of the piercing member 3-30 and the liquid outlet 3-11 according to actual needs, ensuring that the tip portion 3-31 of the piercing member faces the liquid outlet 3-11, thereby being further conducive to piercing the sealing film.

According to another specific embodiment of the present disclosure, the shape of the pipe 3-10 in the present disclosure is not particularly limited, and those skilled in the art can make selections according to actual needs. For example, the pipe 3-10 can be cylindrical or semi-cylindrical, which can further facilitate the pipe forming and simplify the pipe structure.

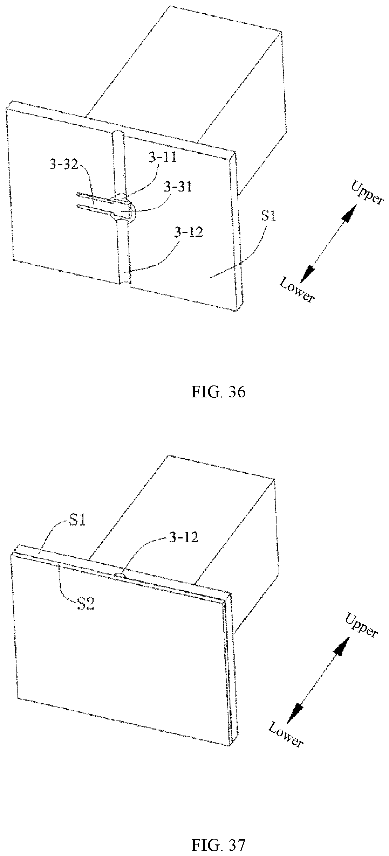

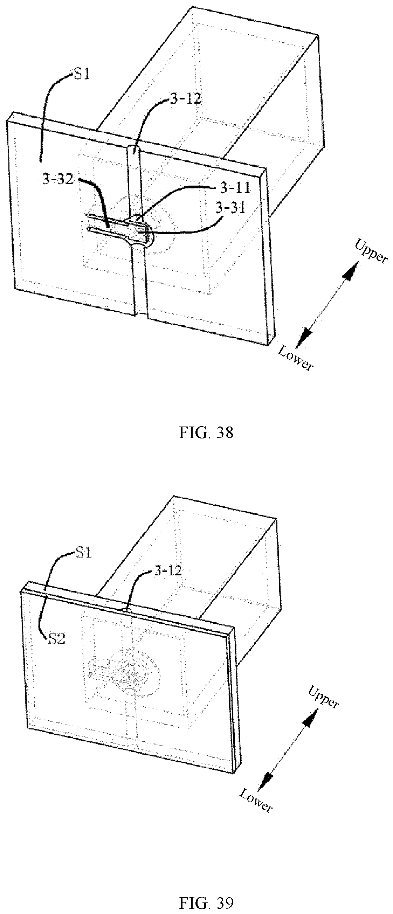

According to another specific embodiment of the present disclosure, as shown in FIGS. 36 and 37, the pipe 3-10 may include a first plate S1 and a second elastic plate S2. A lower surface of the first plate S1 has a groove 3-12, and a liquid outlet 3-11 is provided in the groove 3-12; and the elastic plate S2 is arranged on the lower surface of the first plate S1 and seals the groove 3-12 so that the sealed groove 3-12 forms the microfluidic channel. The piercing member 3-30 is provided in the microfluidic channel and at a position opposed to the liquid outlet 3-11. The piercing member 3-30 is connected to the lower surface of the first plate S1 through an elastic connecting portion 3-32, with the tip portion 3-31 facing the liquid outlet 3-11 (atop view of the piercing member 3-30 is shown in FIGS. 30 and 35), where the elastic connecting portion is an elastic member. The tip portion 3-31 of the piercing member 3-30 pierces through the sealing film 3-20 when the elastic plate S2 is squeezed under an external force, as shown in FIGS. 38 and 39. This not only facilitates the forming of the pipe 3-10, but also facilitates the piercing of the sealing film 3-20. Preferably, the first plate S1 is relatively harder than the elastic plate S2, which can further facilitate the forming of the pipe 3-10. It should be noted that the "lower surface" in the "lower surface of the first plate" in the present disclosure is defined based on the top-to-bottom direction shown in FIGS. 36 to 39.

According to another specific embodiment of the present disclosure, the microfluidic pipe of the above embodiment of the present disclosure can be used in a fully automatic microfluidic kit. The liquid outlet 3-11 can be just facing the sample chamber of the kit, such as a freeze-dried powder chamber or a reagent room. The microfluidic pipe can be configured to store the diluent raw materials or the lysis sample mixture. The pipe can be sealed without external force during the storage process, and when the freeze-dried powder needs to be diluted, it is only required that the sealing film be pierced when an external force is applied to the end portion of the pipe 3-10.

According to an embodiment of the present disclosure, the microfluidic kit chip includes the microfluidic pipe described in the above embodiment of the present disclosure. This can further simplify the structure of the microfluidic kit chip. It should be noted that the technical features and beneficial effects described above for the microfluidic pipe are also applicable to the microfluidic kit chip, and will not be repeated here.

In the description of the specification, descriptions with reference to the terms "an embodiment," "some embodiments," "an example," "a specific example," or "some examples" mean that a particular feature, structure, material, or characteristic described in connection with the embodiment or example are included in at least one embodiment or example of the present disclosure. The schematic representations of the above terms throughout this specification are not necessarily referring to the same embodiment or example of the present disclosure. Furthermore, the described particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of present disclosure have been shown and described above, it should be understood that above embodiments are just illustrative, and cannot be construed to limit the present disclosure, and for those skilled in the art, changes, modifications, replacements, and variations can be made to the embodiments without departing from the scope of the present disclosure.

## Claims

1. A molecular detection system, comprising:
a substrate;
a fixing apparatus;
a driving apparatus;
a detection apparatus; and
a temperature control apparatus,
wherein the fixing apparatus, the driving apparatus, the detection apparatus, and the temperature control apparatus are arranged on the substrate,
the fixing apparatus is adapted to fix a container containing a sample to be detected;
the driving apparatus is adapted to drive the container to preprocess the sample to be detected;
the detection apparatus is adapted to detect the sample to be detected that has undergone the preprocessing; and
the temperature control apparatus is adapted to regulate a temperature of the sample to be detected.

2. The molecular detection system according to claim 1, wherein the substrate comprises a first base, a support, and a shaft sleeve, and the first base, the support, and the shaft sleeve are connected to each other.

3. The molecular detection system according to claim 2, wherein the fixing apparatus comprises a slot, a first motor, a first nut, a first clamping member, a second clamping member and a connecting rod; the first motor is connected to the first clamping member by the first nut, the first clamping member is connected to the second clamping member by the connecting rod, and the slot is provided between the first clamping member and the second clamping member.

4. The molecular detection system according to claim 3, further comprising: a first compression spring disposed between the slot and the second clamping member.

5. The molecular detection system according to claim 3 or 4, further comprising: a first heat dissipation component provided under the slot.

6. The molecular detection system according to claim 2, wherein the driving apparatus comprises a second motor, a third motor, a thimble, and a push rod, the second motor is connected to the thimble, and the third motor is connected to the push rod.

7. The molecular detection system according to claim 6, further comprising: a linear guide rail and a sliding block, wherein the push rod is arranged on the linear guide rail through the sliding block.

8. The molecular detection system according to claim 3, wherein the detection apparatus comprises an optical path detection unit, and the temperature control apparatus comprises a chip temperature cycle control unit.

9. The molecular detection system according to claim 8, wherein one end of the detection apparatus is connected to the slot through the chip temperature cycle control unit.

10. The molecular detection system according to claim 2, further comprising a control system, wherein the control system is connected respectively to the fixing apparatus, the driving apparatus, the detection apparatus, and the temperature control apparatus, and is adapted to control the fixing apparatus, the driving apparatus, the detection apparatus, and the temperature control apparatus to operate collaboratively to detect the sample to be detected.

11. The molecular detection system according to any one of claims 1 to 10, further comprising:
a sliding apparatus connected to the driving apparatus and the fixing apparatus, and adapted to hold the container in a relatively fixed position.

12. The molecular detection system according to claim 11, wherein the sliding apparatus comprises:
a second base;
a first guide rod movably inserted in a fixed limit through hole located on one end of the second base;
a second guide rod movably inserted in a movable limit through hole on the other end of the second base;
a first connecting bracket having a third through hole provided thereon, wherein an upper end of the first guide rod and an upper end of the second guide rod are connected to two ends of the first connecting bracket, respectively;
a second connecting bracket, wherein a lower end of the first guide rod and a lower end of the second guide rod are connected to two ends of the second connecting bracket, respectively; and
a first screw motor arranged on the second base, wherein a screw in the first screw motor is perpendicular to the second base and extends through the third through hole, and the screw is adapted to drive the first connecting bracket to move and jointly move the first guide rod, the second guide rod and the second connecting bracket.

13. The molecular detection system according to claim 12, wherein a diameter of the first guide rod is 0.1mm to 0.3mm smaller than an inner diameter of the fixed limit through hole, and a diameter of the second guide rod is 0.5mm to 2mm smaller than an inner diameter of the movable limit through hole.

14. The molecular detection system according to claim 12, wherein the upper end of the first guide rod and the upper end of the second guide rod are connected to the two ends of the first connecting bracket through bolts, respectively, and the lower end of the first guide rod and the lower end of the second guide rod are connected to the two ends of the second connecting bracket through bolts, respectively.

15. The molecular detection system according to claim 12, wherein the screw is connected to the third through hole through screw threads.

16. The molecular detection system according to claim 15, further comprising:
a first screw nut fixedly connected at the third through hole and matched with and connected to the screw.

17. The molecular detection system according to claim 16, wherein the first connecting bracket is movable within an interval of 5mm to 55mm from the second base.

18. The molecular detection system according to claim 12, wherein a lubricating layer is formed on a surface of each of the fixed limit through hole and the movable limit through hole.

19. The molecular detection system according to claim 18, wherein the lubricating layer is a lubricating oil layer or a graphite layer, and a thickness of the lubricating layer is 0.01mm to 1mm.

20. The molecular detection system according to any one of claims 12 to 19, wherein the screw in the first screw motor is a trapezoidal screw, a ball screw or a planetary ball screw, and a motor in the first screw motor is a direct current motor, a stepper motor or a servo motor.

21. The molecular detection system according to any one of claims 1 to 10, further comprising:
an optical path imaging automatic adjustment apparatus connected to the detection apparatus, and comprising a photoelectric sensor and a convex lens, wherein the optical path imaging automatic adjustment apparatus is adapted to automatically adjust a distance between the photoelectric sensor and the convex lens.

22. The molecular detection system according to claim 21, wherein the optical path imaging automatic adjustment apparatus further comprises:
a third base, wherein the photoelectric sensor is fixed on the third base;
an adjusting bracket movably arranged on the third base,wherein the convex lens is provided on the adjusting bracket, and the distance between the photoelectric sensor and the convex lens is adjusted by moving the adjusting bracket; and
a second screw motor fixed on the third base, wherein a screw in the second screw motor passes through the adjusting bracket and drives the adjusting bracket to move.

23. The molecular detection system according to claim 22, wherein the adjusting bracket is threadedly connected to the screw.

24. The molecular detection system according to claim 23, further comprising:
a second screw nut fixedly connected to the adjusting bracket and matched with and connected to the screw.

25. The molecular detection system according to claim 24, wherein the adjusting bracket is movable within a distance smaller than or equal to 30mm.

26. The molecular detection system according to claim 25, wherein the distance between the photoelectric sensor and the convex lens is 30mm to 60mm.

27. The molecular detection system according to claim 22, wherein a plurality of protrusions that abuts against an inner wall of the third base is provided on a peripheral wall of the adjusting bracket.

28. The molecular detection system according to claim 23, wherein the second screw motor and the photoelectric sensor are intelligently and jointly controlled through a CPU end.

29. The molecular detection system according to claim 23, wherein the screw in the second screw motor is a trapezoidal screw, a ball screw, or a planetary ball screw, and a motor in the second screw motor is a direct current motor, a stepper motor or a servo motor.

30. The molecular detection system according to any one of claims 1 to 29, wherein the slot comprises:
a housing, wherein an upper part of the housing is open and defines an accommodating space inside;
at least one positioning hole defined in a side portion of the housing;
at least one positioning pin each having one end extending into one of the at least one positioning hole;
a heating assembly arranged in the accommodating space; and
a second compression spring arranged at a bottom of the accommodating space and connected to the heating assembly.

31. The molecular detection system according to claim 1, wherein a via hole is defined in a bottom of the housing, the slot further comprises a fixing screw extending through the via hole into the accommodating space to be connected to the heating assembly, and the second compression spring is sleeved on the fixing screw.

32. The molecular detection system according to claim 30, wherein the at least one positioning hole comprises a plurality of positioning holes, the at least one positioning pin comprises a plurality of positioning pins, and the plurality of positioning holes is arranged in one-to-one correspondence with the plurality of positioning pins.

33. The molecular detection system according to claim 30, wherein the end of the positioning pin extending into the positioning hole is conical.

34. The molecular detection system according to claim 31, wherein the heating assembly comprises a heating sheet and a heat conduction block that are connected to each other, and the heat conduction block is connected to the second compression spring and the fixing screw.

35. The molecular detection system according to claim 34, wherein a heating chamber is formed on the heat conduction block.

36. The molecular detection system according to claim 35, wherein a lower part of the heat conduction block is provided with a limiting hole, and the fixing screw extends into the limiting hole.

37. The molecular detection system according to claim 35, wherein a diameter of the via hole is larger than a diameter of the fixing screw.

38. A microfluidic kit adapted for the molecular detection system according to any one of claims 1 to 37, wherein the microfluidic kit comprises:
a box body having a reagent zone, a sample zone, and a reaction zone inside;
a microfluidic kit chip comprising a microfluidic pipe with a piercing member provided inside and facing a liquid outlet, wherein the microfluidic kit chip is connected respectively to the reagent zone, the sample zone and the reaction zone through the microfluidic pipe.

39. The microfluidic kit according to claim 38, wherein the microfluidic pipe further comprises:
a pipe having an end portion made of an elastic material, wherein the liquid outlet is provided in a side wall of the end portion of the pipe; and
a sealing film arranged at the liquid outlet and sealing the liquid outlet;
wherein the piercing member has a tip portion, the piercing member is located on an inner wall of the end portion of the pipe opposed to the liquid outlet, wherein the tip portion faces the liquid outlet and is configured to pierce through the sealing film when the end portion is squeezed under an external force.

40. The microfluidic kit according to claim 39, wherein the piercing member and the liquid outlet are provided on a same diameter of the pipe.

41. The microfluidic kit according to claim 39, wherein the sealing film is a tin foil paper, a laminating film or a kraft paper.

42. The microfluidic kit according to claim 39, wherein the sealing film has a thickness in a range from 0.01mm to 0.2mm.

43. The microfluidic kit according to claim 39, wherein a tip of the tip portion has a tapered structure, the tapered structure being a cone or a polygonal pyramid.

44. The microfluidic kit according to claim 39, wherein the tip portion is formed by a triangular surface, wherein one side of the triangular surface is provided on the inner wall of the end portion of the pipe opposed to the liquid outlet and an apex angle corresponding to the side of the triangular surface forms a tip of the tip portion.

45. The microfluidic kit according to claim 44, wherein the side of the triangular face provided on the inner wall of the end portion of the pipe is straight line-shaped, V-shaped, arc-shaped or U-shaped.

46. The microfluidic kit according to claim 39, wherein the piercing member and the pipe are integrally formed into one piece.

47. The microfluidic kit according to claim 39, wherein the pipe further comprises:
a first plate, wherein a lower surface of the first plate has a groove, and the liquid outlet is located in the groove; and
an elastic plate arranged on the lower surface of the first plate and sealing the groove so that the groove forms a microfluidic channel,
wherein the piercing member is located in the microfluidic channel and at a position opposed to the liquid outlet, the piercing member is connected to the lower surface of the first plate through an elastic member, and the piercing member is configured to pierce through the sealing film when the elastic plate is squeezed under an external force.
